# EUROPÄISCHE PATENTSCHRIFT

(11) **EP 1 474 415 B1**
(45) Veröffentlichungstag und Bekanntmachung des Hinweises auf die Patenterteilung: **27.07.2011**
(21) Anmeldenummer: 03702464.3
(22) Anmeldetag: 17.01.2003
(51) Int. Cl.: C07D 401/04, C07D 409/04, C07D 413/14, C07D 417/14, A61K 31/415

(54) **1-PHENYL-2-HETEROARYL-SUBSTITUIERTE BENZIMIDAZOLDERIVATE, DEREN VERWENDUNG ZUR HERSTELLUNG VON ARZNEIMITTELN ZUR BEHANDLUNG VON IMMUNOLOGISCHEN ERKRANKUNGEN**
1-PHENYL-2-HETEROARYL-SUBSTITUTED BENZIMIDAZOLE DERIVATIVES, THE USE THEREOF FOR PRODUCING DRUGS USED IN THE TREATMENT OF IMMUNOLOGICAL DISEASES
DERIVES DE BENZIMIDAZOLE 1-PHENYL-2-HETEROARYL SUBSTITUES ET LEUR UTILISATION POUR PRODUIRE DES MEDICAMENTS SERVANT A TRAITER DES MALADIES IMMUNOLOGIQUES

(30) Priorität: 15.02.2002 DE 10207844
(43) Veröffentlichungstag der Anmeldung: 10.11.2004
(73) Patentinhaber: Bayer Schering Pharma Aktiengesellschaft, 13353 Berlin (DE)
(72) Erfinder: BLUME, Thorsten, 16552 Schildow (DE); HALFBRODT, Wolfgang, 13505 Berlin (DE); KUHNKE, Joachim, 14469 Potsdam (DE); MÖNNING, Ursula, 15569 Woltersdorf (DE); ELGER, Bernd, 13505 Berlin (DE); SCHNEIDER, Herbert, 14129 Berlin (DE)
(86) Internationale Anmeldenummer: PCT/EP2003/000462
(87) Internationale Veröffentlichungsnummer: WO 2003/068766

(56) Entgegenhaltungen:
- WO-A-01/51473

## Beschreibung

Die Erfindung betrifft neue Benzimidazolderivate, deren Herstellung und deren Verwendung zur Herstellung von Arzneimitteln zur Behandlung und Prophylaxe von Erkrankungen, die mit einer Mikroglia-Aktivierung assoziiert sind, und von T-Zell-vermittelten immunologischen Erkrankungen, sowie pharmazeutische Präparate, die die neuen Benzimidazolderivate enthalten.

Das Immunsystem umfasst eine Vielzahl von Zellen und Gewebekomplexen, die hauptsächlich über lösliche Faktoren miteinander kommunizieren. Es ist bekannt, dass viele immunologische Erkrankungen durch eine Inbalance von löslichen Immunfaktoren, wie z. B. den Cytokinen, ausgelöst werden (Mosmann and Coffmann., Ann. Rev. Immunol. 7: 145 - 173 (1989Street and Mosmann, FASEB J. 5: 171 - 177 (1991); Lucey et al., Clin. Microbiol. Rev. 4: 532 - 562 (1996); Powrie and Coffman, Trends Pharmacol. Sci 14: 164 - 168 (1993); Singh et al., Immunolog. Res., 20: 164-168 (1999)). So gibt es zum Beispiel eine Vielzahl von Hinweisen auf eine Rolle von Interferon gamma und Interleukin 12 in der Pathogenese von Autoimmun-Erkrankungen. Speziell zu nennen sind Erkrankungen, die durch eine T-Zell-vermittelte inflammatorische Reaktion gekennzeichnet sind, wie die Multiple Sklerose, Diabetes, chronische inflammatorische Darm-Erkrankungen (Inflammatory Bowel Diseases). Das Cytokin Interleukin 12 (IL 12) wird von phagozytären Zellen, wie Makrophagen/Monozyten, Dendriten, B-Zellen und anderen Antigenpräsentierenden Zellen (APC), produziert und beeinflusst sowohl die Funktion von natürlichen Killerzellen (NK-Zellen) als auch die von T-Lymphozyten. IL 12 kann in beiden Zelltypen die Produktion von Interferon gamma (IFNγ) anregen. T-Lymphozyten kann man grob in zwei Kategorien einteilen, die durch die Expression von bestimmten Oberflächen-Antigenen (CD4 und CD8) charakterisiert sind: CD4-positive T-Zellen (Helfer-T-Zellen) und CD8-positive T-Zellen (zytotoxische T-Zellen). Die CD4-Zellen können wiederum in T-Helfer-Zellen 1 (Th1) und T-Helfer-Zellen 2 (Th2) unterteilt werden. Speziell die Th1-vermittelten Immunantworten sind mit der Pathogenese zahlreicher Immunerkrankungen, speziell den Autoimmun-Erkrankungen, assoziiert, wie z.B.: Typ I Insulin-abhängiger Diabetes Mellitus (IDDM), Multiple Sklerose, Allergisches Kontaktekzem, Psoriasis, Rheumatoide Arthritis, chronische inflammatorische Darmerkrankungen ("Inflammatory Bowel diseases" - Morbus Crohn, Colitis ulcerosa), Lupus-Erkrankungen und andere Kollagenosen sowie akute -Abstoßungsreaktionen in der Allotransplantation ("Host-versus-Graft "-Allotransplantatabstossung, "Graft-versus-host Disease").

Von Interleukin 12 ist bekannt, dass es eine kritische Rolle in der Regulation der Th1-Antwort spielt. Interleukin 12 induziert in diesen Zellen die Produktion von hauptsächlich IL-2, IFNγ, TNFα und TNFβ (Mosmann and Sad, Immunol. Today 17: 138 - 146 (1996) ; Gately et al., Annu. Rev. Immunol. 16: 495 - 521 (1998)). Speziell IFNγ ist ein potenter Vermittler der IL-12-Wirkung. Eine Überproduktion von Interferon gamma kann beispielsweise für die MHC II (Major Histocompatibility Complex)-assoziierten Autoimmunerkrankungen verantwortlich sein (. Zusätzlich gibt es auch genügend Evidenzen hinsichtlich einer pathologischen Rolle von Interferon gamma in allergischen Erkrankungen sowie der Sarcoidosis und der Psoriasis. (Billiau A., Adv. Immunol., 62: 61-130 (1996); Basham et al. J. Immunol. 130: 1492 - 1494 (1983); Hu et al., Immunology, 98: 379-385 (1999); Seery JP., Arthritis Res., 2: 437-440 (2000)). Darüberhinaus sind IL-12 und IL-12/IL-18-induziertes IFNγ aus NK-Zellen wesentlich in den Pathomechanismus nicht T-zellvermittelter Entzündungsreaktionen (z.B. "Toxic Shock Syndrome", Endotoxinämie, Sepsis und Septischer Schock, ARDS, "first dose response" bei Antikörpertherapie z.B. OKT3-Gabe bei Allotransplantation) beteiligt (Kum et al., Infect Immun. 69: 7544-7549 (2001); Arad et al., J Leukoc. Biol. 69: 921-927 (2001); Hultgren et al., Arthritis Res. 3: 41-47 (2001), Arndt et al., Am. J. Respir. Cell. Mol. Biol. 22: 708-713 (2000); Grohmann et al., J. Immunol. 164: 4197-4203 (2000); Muraille et al., Int. Immunol. 11: 1403-1410 (1999)). IL-12 spielt ebenfalls eine Rolle bei Entzündungen mit derzeit unklaren Pathomechanismen (z.B. Eklampsie)

(Hayakawa et al., J. Reprod. Immunol. 47: 121-138 (2000); Daniel et al., Am. J. Reprod. Immunol. 39: 376-380 (1998)).

Neben Interleukin 12 und IFNγ wird noch anderen Cytokinen eine Rolle in der Pathogenese von Immunerkrankungen und systemischen Entzündungsreaktionen zugeschrieben, wie beispielsweise dem TNFα. TNFα spielt eine bedeutende pathologische Rolle bei Infektionserkrankungen (wie Sepsis, "toxic shock syndrome" (Tracey et al., Nature 330: 662 - 664 (1987); Basger et al., Circ. Shock, 27: 51 - 61 (1989); Hinshaw et al., Circ. Shock, 30: 279 - 292 (1990); Waage A., Lancet, 351: 603 (1998); Cohen et al., Lancet, 351: 1731 (1998)), aber auch zahlreichen anderen immun-vermittelten Erkrankungen.

Für die Behandlung von IL 12-vermittelten Erkrankungen und für die Verminderung der akuten Symptome dieser Erkrankungen werden häufig Corticosteroide eingesetzt, deren Nebenwirkungen insbesondere bei Langzeitbehandlung häufig zum Abbruch der Behandlung führen.

Die Aktivierung der Mikroglia stellt einen zentralen Schritt im Entzündungsgeschehen von nahezu allen degenerativen Erkrankungen des Zentralen Nervensystems dar. Die Mikroglia können über einen längeren Zeitraum in dem aktivierten Zustand bleiben, in dem sie verschiedene Entzündungsfaktoren, z. B. reaktive Sauerstoff/Stickstoff-Intermediate, Proteasen, Cytokine, Komplement-Faktoren und Neurotoxine, produzieren und sekretieren. Diese wiederum bewirken neuronale Dysfunktion und Degeneration. Die Aktivierung von Mikroglia kann durch verschiedene Stimuli erfolgen, wie z. B. Aß-Peptid (β-Amyloid, Araujo, D.M. and Cotman, C.M., Brain Res. 569: 141 - 145 (1992)), Prion-Protein, Cytokine oder durch Zellfragmente (Combs, C. K. et al., J. Neurosci. 19: 928 - 939 (1999); Wood, P. L., Neuroinflammation: Mechanisms and Management, Humana Press (1998).

Benzimidazole, die nach Stimulierung mit dem Aß-Peptid die Aktivierung der Mikroglia hemmen, werden in WO 01/51473 beschrieben. Daraus ist auch bekannt, dass Benzimidazole, die die Aktivierung der Mikroglia hemmen, zur Behandlung neuroinflammatorischer Erkrankungen, wie AIDS-Dementia, Amyotrophe Lateralsklerose, Creutzfeldt-Jakob-Krankheit, Down's Syndrome, diffuse Lewy Body Krankheit, Huntington's Krankheit, Leukencephalopathy, Multiple Sklerose, Parkinsonsche Krankheit, Picksche Krankheit, Alzheimersche Krankheit, Schlaganfall, temporäre Lobe-Epilepsie und von Tumoren, eingesetzt werden.

EP 0 104 727 A1 beschreibt Benzimidazolderivate, die in 1-Stellung nicht substituiert sind und in 2-Stellung eine Alkylgruppe aufweisen. Substituenten am Benzolring der Derivate sind u.a. Pyridyloxy-, Pyridylalkyl-, Pyridylalkyloxy- und Pyridyloxyalkandiyl-Reste.

In WO 01/21634 A1 sind ferner Benzimidazolderivate beschrieben, die in 1-Stellung eine Alkandiylamidogruppe, in 2-Stellung u.a. einen substituierten Phenyl- oder Heteroaryl-Rest und am anellierten Benzolring u.a. mindestens mit einem substituierten Alkoxy-, Alkylamino-, Alkylsulfonyl- und Alkylsulfoxid-Rest substituiert sein können. Es wird angegeben, dass diese Substanzen für eine Vielzahl möglicher Indikationen als Wirkstoff in Arzneimittelzubereitungen eingesetzt werden können.

In US-A-5,552,426 sind substituierte Benzimidazole angegeben, die in 1-Stellung u.a. einen Phenyl- oder Naphthylrest und in 2-Stellung u.a. einen Phenyl- oder Heterocyclusrest aufweisen. Der anellierte Benzolring der Benzimidazole ist vorzugsweise mit einem Alkoxy- oder Aminoalkoxyrest substituiert. Derartigen Verbindungen wird eine Wirksamkeit gegen Erkrankungen zugeschrieben, die auf einer mit einem β-Amyloid-Peptid assoziierten Neurotoxizität beruhen.

In WO 97/12613 A1 sind verschiedene entzündungshemmende und Artheriosklerose verhindernde Mittel beschrieben. Beispielsweise werden Benzimidazolderivate als Wirkstoffe angegeben, die in 1-Stellung u.a. mit einem Phenyl- oder substituierten Phenylrest und in 2-Stellung mit einem Alkoxyrest substituiert sind. Substituenten am Benzolring der Wirkstoffverbindungen können u.a. Alkyl-, Nitro-, Halogeno-, Alkoxy-, Amino-, Ester-, Amid-, Alkandiylalkoxy- und Alkandiylaminoreste sein.

In EP 0 520 200 A2 sind Benzimidazolderivate angegeben, die in 1-Stellung substituierte Arylreste und in 2-Stellung einfach-, zweifach-substituierte oder unsubstituierte Aminogruppen aufweisen. Der Benzolring des Benzimidazolgrundgerüsts kann mit Halogen, Trifluormethyl und/oder Cyano substituiert sein. Diese Verbindungen dienen zur Behandlung von Erkrankungen, die mit einer verstärkten Aktivierung von Ca-Kanälen verbunden sind.

In WO 97/33873 A1 sind ebenfalls Benzimidazolderivate angegeben, die zur Behandlung von Zystitis eingesetzt werden. Diese Verbindungen können in 1-Stellung u.a. Phenyl-, Naphthyl- und ungesättigte Heterocyclusreste aufweisen. In 2-Stellung können die Derivate mit Alkoxy-, Phenylalkoxy-, Naphthylalkoxy-, Heterocyclusalkoxy- oder ungesättigten Heterocyclusalkoxyresten substituiert sein. Der Benzolring des Grundgerüstes der Derivate kann mit Nitro-, Alkanoyl-, Amino-, Alkyl-, Alkoxy-, Cycloalkyl-, Heterocyclus-, ungesättigten Heterocyclus-, Halogeno-, Alkylthio-, Hydroxyalkylidenyl-, Hydroxyalkylidenylamino-, Aminoalkylidenyl-, Aminoalkoxy-, Hydroxyalkyl-, Heterocyclusalkoxy-, Aminoalkylidenyl- oder Trifluormethylresten substituiert sein.

In EP 0 531 883 A1 sind kondensierte 5-gliedrige Heterocyclen angegeben, beispielsweise substituierte Benzimidazolderivate, wobei diese Verbindungen gemäß der allgemeinen Beschreibung der Verbindungen in 1-Stellung vorzugsweise mit einem substituierten Alkylrest und in 2-Stellung beispielsweise mit einem O-Alkandiyl-, S-Alkandiyl-, NH-Alkandiyl-, N(Alkyl)-Alkandiyl-, SO-Alkandiyl- oder SO₂-Alkandiylrest substituiert sind. Die beschriebenen Verbindungen sollen antithrombische Wirksamkeit aufweisen.

Für eine mögliche Therapie der Neuroinflammation sind bisher nicht-steroidale Entzündungshemmer (COX II Inhibitoren) [McGeer, P.L., Roger, Neurology, 42, 447-449 (1992), Rogers, J., Kirby, L.C., Hempleman, S.R., Berry, D.L., McGeer, P.L., Kaszniak, A.W., Zalinski, J., Cofield, M., Mansukhani, L., Wilson, P., Kogan, F., Neurology, 43, 1609-1611 (1993), Andersen, K., Launer, L.J., Ott, A., Hoes, A.W., Breteler, M.M.B., Hofman, A., Neurology, 45, 1441-1445 (1995), Breitner, J.C.S., Gau, B.A., Welsh, K.A., Plassman, B.L., McDonald, W.M., Helms, M.J., Anthony, J.C., Neurology, 44, 227-232 (1994), The Canadian Study of Health and Aging, Neurology, 44, 2073-2079 (1994)), Cytokin-Modulatoren [McGeer, P.L., McGeer, E.G., Brain Res. Rev., 21: 195-218 (1995), McGeer, E.G., McGeer, P.L., CNS Drugs, 7, 214-228 (1997), Barone, F.C. and Feuerstein, G.Z, J. Cerebral Blood Flow and Metabolism, 19, 819-834 (1999)] und Komplement-Kaskaden-Inhibitoren [Chen, S., Frederickson, R.C.A., and Brunden, K.R., Neurobiol. Aging (1996), McGeer, E.G., McGeer, P.L, Drugs, 55: 739-746 (1998)] beschrieben worden.

Die zur Therapie immunologischer Erkrankungen bekannten Verbindungen, wie z. B. Steroide, wirken häufig auf mehrere Faktoren im Immunsystem und lösen dadurch zahlreiche Nebenwirkungen aus. Es besteht daher die Aufgabe Substanzen zur Verfügung zu stellen, die auf Grund ihrer Mikroglia-Aktivität die Cytokin-Aktivität hemmen, ohne gravierende toxische Nebenwirkungen auszulösen.

Das Problem wird gelöst durch neuartige Benzimidazolderivate gemäß Anspruch 1, weiterhin durch die Verwendung eines erfindungsgemäßen Benzimidazolderivates zur Herstellung eines Arzneimittels zur Unterbrechung der IL 12 und INFγ-Produktion in Zellen monozytären Ursprungs bzw. T-Zellen und NK-Zellen.

Auf Grund ihrer Fähigkeit, die Produktion von IL 12 und TNFα in Monozyten/Makrophagen/Dendriten und die IFNγ Produktion in T-Zellen und NK-Zellen zu unterbrechen, sind Mikroglia-Inhibitoren zur Behandlung zahlreicher Erkrankungen geeignet, die durch die verstärkte Produktion von Cytokinen, wie z.B. TNFα,β, IFNγ, IL-2 und IL12 ausgelöst werden, wie inflammatorische Erkrankungen, die nicht auf Neuroinflammation beruhen, Autoimmun-Erkrankungen, allergische und infektiöse Erkrankungen, Toxin-induzierte Entzündungen, pharmakologisch ausgelöste Entzündungsreaktionen sowie pathophysiologisch relevante Entzündungsreaktionen derzeit unklarer Genese.

Die erfindungsgemäßen Benzimidazolderivate weisen folgende allgemeine Strukturformel I auf:

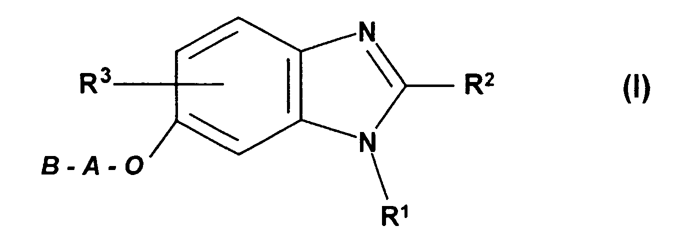

Darin sind:
- ***R¹***: eine Phenylgruppe, die gegebenenfalls mit bis zu drei folgenden Substituenten unabhängig voneinander substituiert ist:
F, Cl, Br, J
OH, OR⁴, OCOR⁴
SR⁴, SOR⁴, SO₂R⁴,
R⁴,
NH₂, NHR⁴, NR⁴R^{4,}
oder zwei benachbarte Substituenten an ***R¹*** bilden zusammen eine -O-CH₂-O-, -O-CH₂-CH₂-O- oder -CH₂-CH₂-CH₂- Gruppe,
- ***R²***: eine monocyclische oder bicyclische 5 - 10-gliedrige Heteroarylgruppe mit 1 - 2 Heteroatomen ausgewählt aus N, S und O, die gegebenenfalls mit bis zu zwei der folgenden Substituenten unabhängig voneinander substituiert ist:

F, Cl, Br, J
OH, OR⁴, OCOR⁴,
COR⁴,
SR⁴, SOR⁴, SO2R⁴,
R⁴
oder zwei benachbarte Substituenten an R² bilden zusammen eine -O-CH₂-O-, -O-CH₂-CH₂-O- oder -CH₂-CH₂-CH₂- Gruppe,
- ***R³***: H, OH oder O-C₁₋₆-Alkyl,
- ***R⁴*** und ***R^{4,}***: unabhängig voneinander C₁₋₄-Perfluoralkyl oder C₁₋₆-Alkyl,
- ***A***: eine C₂₋₆-geradkettige Alkylengruppe, ausgewählt aus Ethylen, Propylen und Butylen, die gegebenenfalls mit =O, OH, O-C₁₋₃-Alkyl, NH₂, NH-C₁₋₃-Alkyl, NH-C₁₋₃-Alkanoyl, N(C₁₋₃-Alkyl)₂, und N(C₁₋₃-Alkyl)(C₁₋₃-Alkanoyl) substituiert ist,
- ***B***: COOH, CONH₂, CONHNH₂, CONHR⁵, CONR⁵R^{5'}, jeweils gebunden an ein Kohlenstoffatom der Gruppe A,
- ***R⁵*** und ***R^{5'}***: unabhängig voneinander jeweils ein Rest, ausgewählt aus der Gruppe umfassend C₁₋₆-Alkyl, C₂₋₆-Alkenyl, C₂₋₆-Alkinyl, wobei ein C-Atom gegen O, S, SO, SO₂, NH, N-C₁₋₃-Alkyl oder N-C₁₋₃-Akaloyl ausgetauscht sein kann, ferner (C₀₋₃-Alkandiyl-C₃₋₇- Cycloalkyl), wobei in einem fünfgliedrigen Cycloalkylring ein Ringglied Ring-N oder Ring-O sein kann und in einem sechs- oder siebengliedrigen Cycloalkylring ein oder zwei Ringglieder jeweils Ring-N- und/oder Ring-O-Atome sein können, wobei die Ring-N- Atome gegebenenfalls mit C₁₋₃-Alkyl oder C₁₋₃-Alkanoyl substituiert sein können, sowie ferner (C₀₋₃-Alkandiyl-Phenyl) und (C₀₋₃-Alkandiyl-Heteroaryl), wobei die Heteroarylgruppe fünf- oder sechsgliedrig ist und ein oder zwei Heteroatome enthält, ausgewählt aus der Gruppe umfassend N, S und O,
wobei alle zuvor genannten Alkyl- und Cycloalkylreste gebenenfalls mit bis zu zwei Resten, ausgewählt aus der Gruppe umfassend CF₃, C₂F₅, OH, O-C₁₋₃-Alkyl, NH₂, NH-C₁₋₃-Alkyl, NH-C₁₋₃-Alkanoyl, N(C₁₋ ₃-Alkyl)₂, N(C₁₋₃-Alkyl)(C₁₋₃-Alkanoyl), COOH, CONH₂ und COO-C₁₋₃- Alkyl, und alle zuvor genannten Phenyl- und Heteroarylgruppen, gegebenenfalls mit bis zu zwei Resten, ausgewählt aus der Gruppe umfassend F, Cl, Br, CH₃, C₂H₅, OH, OCH₃, OC₂H₅, NO₂, N(CH₃)₂, CF₃, C₂F₅ und SO₂NH₂ substituiert sein können
oder R⁵ und R^{5'} gemeinsam mit dem N-Atom einen fünf- bis siebengliedrigen heterocyclischen Ring bilden, der ein weiteres N- oder O- oder S-Atom enthalten kann und der substituiert sein kann mit C₁₋₄-Alkyl, (C₀₋₂-Atkandiyl-C₁₋₄-Alkoxy), C₁₋₄-Alkoxycarbonyl, Aminocarbonyl oder Phenyl

sowie deren optische oder geometrische isomere oder tautomere Formen oder pharmazeutisch anwendbare Salze.

Bevorzugt sind Verbindungen, worin
- ***R¹***: eine Phenylgruppe ist, die gegebenenfalls mit bis zu zwei der folgenden Substituenten unabhängig voneinander substituiert ist:
F, Cl,
OH, OR⁴, OCOR⁴
SR⁴,
R⁴ oder
zwei benahbarte Substituenten an ***R¹*** bilden eine -O-CH₂-O- oder - CH₂-CH₂-CH₂-Gruppe.

Bevorzugt sind auch Benzimidazolderivate, bei denen
- ***R²***: eine monocyclische 5-6 gliedrige Heteroarylgruppe mit 1-2 Heteroatomen ausgewählt aus der Gruppe umfassend N, S und O ist, die gegebenenfalls mit bis zu zwei der folgenden Substituenten unabhängig substituiert ist:
F, Cl,
OR⁴, OCOR⁴
SR⁴, SOR⁴,SO₂R⁴,
R⁴ oder
zwei benachbarte Substituenten an ***R²*** bilden eine -O-CH₂-O- oder CH₂-CH₂-CH₂-Gruppe.

Bevorzugt sind auch Benzimidazolderivate, bei denen
- *R³*: H ist.

Bevorzugt sind auch Benzimidazolderivate, bei denen
- ***R⁴*** und ***R^{4,}***: unabhängig voneinander C₁₋₂ Perfluoralkyl, C₁₋₄ Alkyl sind.

Bevorzugt sind auch Benzimidazolderivate, bei denen
- ***R⁵*** und ***R^{5,}***: unabhängig voneinander C₁₋₆ Alkyl, wobei ein Kohlenstoffatom gegen O, S, SO, SO² ausgetauscht sein kann, C₃₋₅ Cycloalkyl-C₀₋₃ Alkylen, wobei in einem 5-gliedrigen Cycloalkylring ein Ringglied ein N oder ein O sein kann, wobei der Ringstickstoff gegebenenfalls mit C₁₋₃ Alkyl oder C₁₋₃ Alkanoyl substituiert ist,
C₀₋₂ Alkylen-(5-6 gliedriges Heteroaryl mit 1-2 Heteroatomen aus N, S und O)
wobei alle zuvor genannten Alkyl- und Cycloalkylreste mit CF₃, OH, NH2, NH C₁₋₃ Alkyl, NH C₁₋₃ Alkanoyl, N(C₁₋₃Alkyl)₂, N(C₁₋₃Alkyl)(C₁₋₃ Alkanoyl), COOH,CONH₂ und alle zuvor genannten Heteroarylgruppen mit ein oder zwei Substituenten aus der Gruppe bestehend aus F, Cl, CH₃, C₂H₅, OCH₃, OC₂H₅, CF₃, C₂F₅ substituiert sein können,
oder R⁵ und R^{5'} gemeinsam mit dem Stickstoffatom einen 5-7 gliedrigen Heterocyclus bilden, der ein weiteres Sauerstoff-, Stickstoff-
oder Schwefelatom enthalten kann und substituiert sein kann mit C₁₋ ₄-Alkyl, C₁₋₄-Alkoxy-C₀₋₂-alkyl.

Bevorzugt sind Benzimidazolderivate, bei denen
- ***B***: COOH oder CONH₂
jeweils gebunden an ein Kohlenstoffatom der Gruppe A ist.

Besonders bevorzugt sind die folgenden Benzimidazole:
5-[[1-(4-Methylphenyl)-2-(3-pyridinyl)-1*H*-benzimidazol-6-yl)oxy]pentansäure
4-[[1-(4-Methylphenyl)-2-(3-pyridinyl)-1*H*-benzimidazol-6-yl]oxy]buttersäure
5-[[1-(4-Methylphenyl)-2-(3-thienyl)-1*H*-benzimidazol-6-yl]oxy]pentansäure
4-[[1-(4-Methylphenyl)-2-(3-thienyl)-1*H*-benzimidazol-6-yl]oxy]buttersäure
5-[[1-Phenyl-2-(3-thienyl)-1*H*-benzimidazol-6-yl]oxy]pentansäure
4-[[1-Phenyl-2-(3-thienyl)-1*H*-benzimidazol-6-yl]oxy]buttersäure
5-[[1-(4-Fluorphenyl)-2-(3-thienyl)-1*H*-benzimidazol-6-yl]oxy]pentansäure
5-[[1-(4-Fluorphenyl)-2-(3- pyridinyl)-1*H*-benzimidazol-6-yl]oxy]pentansäure
5-[[1-Phenyl-2-(3-pyridinyl)-1*H*-benzimidazol-6-yl]oxy]pentansäure
4-[[1-Phenyl-2-(3-pyridinyl)-1*H*-benzimidazol-6-yl]oxy]buttersäure *N*-(2-Methoxyethyl)-5-[[1-(4-methylphenyl)-2-(3-pyridinyl)-1*H*-benzimidazol-6-yl]oxy]pentanamid
*N,N*-Dimethyl-5-[[1-(4-methylphenyl)-2-(3-pyridinyl)-1*H*-benzimidazol-6-yl]oxy] pentanamid
5-[[1-(4-Methylphenyl)-2-(3-pyridinyl)-1*H*-benzimidazol-6-yl]oxy]pentanamid.

Die vorliegende Erfindung umfasst die physiologisch verträglichen Salze der vorgenannten Verbindungen, insbesondere die Säuresalze der Stickstoffbasen der erfindungsgemäßen Benzimidazolderivate, ferner die Salze von Carbonsäuren der erfindungsgemäßen Derivate mit Basen.

Die erfindungsgemäßen Benzimidazolderivate können ein oder mehrere asymetrische Zentren aufweisen, so dass die Verbindungen in mehreren isomeren Formen auftreten können. Die Verbindungen der Formel I können auch als Tautomere, Stereoisomere oder geometrische Isomere vorliegen. Die Erfindung umfasst auch alle möglichen Isomeren, wie E- und Z-Isomere, S- und R-Enantiomere, Diastereomere, Razemate und Gemische derselben einschliesslich der tautomeren Verbindungen. Alle diese isomeren Verbindungen sind - auch wenn jeweils nicht ausdrücklich angegeben - Bestandteil der vorliegenden Erfindung.

Die Isomerengemische können nach üblichen Methoden, wie beispielsweise Kristallisation, Chromatographie oder Salzbildung, in die Enantiomeren bzw. E/Z-Isomeren aufgetrennt werden.

Die in den erfindungsgemäßen Benzimidazol-Verbindungen enthaltenen Heteroarylgruppen sind aus fünf oder zehn Gerüstatomen aufgebaut und können ein oder zwei Heteroatome enthalten. Heteroatome sind Sauerstoff (O), Stickstoff (N) und Schwefel (S).

Beispiele für monocyclische Heteroarylgruppen sind Pyrrolyl, Thienyl, Furanyl, Imidazolyl, Thiazolyl, Isothiazolyl, Oxazolyl, Isoxazolyl, Pyrazolyl, Pyridyl, Pyrimidinyl, Pyrazinyl und Pyridazinyl.

Beispiele für eine bicyclische Heteroarylgruppe sind Indolyl, Isoindolyl, Benzothiophenyl, Benzofuranyl, Benzimidazolyl, Indazolyl, Chinolyl, Isochinolyl, Phthalazinyl, Chinazolinyl, Cinnolinyl, Chinoxalinyl, Naphthyridinyl. Wenn die Heteroarylgruppe Teil von R¹ ist, erfolgt die Bindung zum N des Benzimidazoles über ein Kohlenstoffatom.

Heteroarylreste können in beliebiger Weise an das Benzimidazol-Grundgerüst oder eine andere Gruppe gebunden sein, beispielsweise als 2-, 3- oder 4-Pyridinyl, als 2- oder 3-Thienyl oder als 1-Imidazolyl.

Alkylgruppen können geradkettig oder verzweigt sein. Beispiele für Alkylgruppen sind Methyl, Ethyl, *n*-Propyl, *iso-*Propyl, *n*-Butyl, *sek*-Butyl, *tert*-Butyl, *n*-Pentyl, *sek*-Pentyl, *tert*-Pentyl, *neo-*Pentyl, *n*-Hexyl, *sek*-Hexyl.

Perfluorierte Alkyle sind vorzugsweise CF₃ und C₂F₅.

Unter Cycloalkylgruppen sind jeweils vorzugsweise Cyclopropyl, Cyclobutyl, Cyclopentyl, Cyclohexyl oder Cycloheptyl zu verstehen.

Als gesättigter heterocyclischer Ring bzw. als Cycloalkyl mit einem oder mehreren Heteroatomen werden beispielsweise genannt: Piperidin, Pyrrolidin, Tetrahydrofuran, Morpholin, Piperazin, Hexahydroazepin sowie 2,6-Dimethylmorpholin, N-Phenyl-piperazin, 2-Methoxymethyl-pyrrolidin, Piperidin-4-carbonamid, Thiomorpholin, Thiazolidin, wobei die Verknüpfung über gegebenenfalls vorhandene Ring-N-Atome erfolgen kann.

A kann zweifach, vorzugsweise einfach, substituiert sein mit OH, NH₂, NH-C₁₋₃-Alkyl oder NH-C₁₋₃-Alkanoyl.

Die physiologisch verträglichen Säuresalze der Stickstoffbasen der erfindungsgemäßen Benzimidazolderivate können mit anorganischen und organischen Säuren gebildet werden, beispielsweise mit Oxalsäure, Milchsäure, Citronensäure, Fumarsäure, Essigsäure, Maleinsäure, Weinsäure, Phosphorsäure, Salzsäure, Bromwasserstoffsäure, Schwefelsäure, p-Toluolsulfonsäure und Methansulfonsäure.

Zur Salzbildung von Säuregruppen, insbesondere Carbonsäuregruppen, sind auch die anorganischen oder organischen Basen geeignet, die zur Bildung physiologisch verträglicher Salze bekannt sind, wie beispielsweise Alkalihydroxide, insbesondere Natrium- und Kaliumhydroxid, Erdalkalihydroxide, wie Calciumhydroxid, ferner Ammoniak, sowie Amine, wie Ethanolamin, Diethanolamin, Triethanolamin, N-Methylglucamin und Tris-(hydroxymethyl)-methylamin.

Die Verbindungen der Formel I inhibieren die Aktivierung der Mikroglia und die Produktion von Interleukin 12 (IL 12) und Interferon γ (IFNγ). Die Erfindung betrifft daher auch die Verwendung einer Verbindung der Formel I, sowie deren optischer oder geometrischer Isomere oder deren Tautomere oder physiologisch verträglichen Salzes zur Herstellung eines Arzneimittels zur Behandlung oder Prevention einer mit einer Mikroglia-Aktivierung assoziierten Erkrankung insbesondere einer durch Überproduktion von IL 12 und IFNγ ausgelösten Erkrankung und zur Induktion von Interleukin 10 (IL-10).

In einer bevorzugten Ausführungsform finden die erfindungsgemäßen Verbindungen Verwendung zur Behandlung einer T-Zell-vermittelten, speziell Th-1-Zell-vermittelten, immunologischen Erkrankung und von nicht T-Zell-vermittelten Entzündungsreaktionen. Insbesondere werden die erfindungsgemäßen Verbindungen verwendet zur Herstellung eines Arzneimittels zur Unterbrechung der IL 12 und INFγ Produktion in Zellen monozytären Ursprungs bzw. T-Zellen und NK-Zellen. Auf Grund ihrer Fähigkeit, die Produktion von IL 12 und TNFα in Monozyten/Makrophagen und die INFγ Produktion in T-Zellen zu unterbrechen, sind die erfindungsgemäßen Verbindungen zur Behandlung zahlreicher Erkrankungen geeignet, die durch die verstärkte Produktion von Cytokinen, wie z.B. TNFα,β, IFNγ, IL 2 und IL 12 ausgelöst werden, wie inflammatorische Erkrankungen, die nicht auf Neuroinflammation beruhen, Autoimmun-Erkrankungen, allergische und infektiöse Erkrankungen, Toxin-induzierte Entzündungen, pharmakologisch ausgelöste Entzündungsreaktionen sowie pathophysiologisch relevante Entzündungsreaktionen derzeit unklarer Genese.

Beispiele für inflammatorische und autoimmune Erkrankungen sind: chronische inflammatorische Darm-Erkrankungen (Inflammatory Bowel Diseases, Crohn's Disease, Ulcerative Colitis), Arthritis, allergisches Kontaktekzem, Psoriasis, Pemphigus, Asthma, Multiple Sklerose, Diabetes, Typ-I Insulin-abhängiger Diabetes Mellitus, Rheumatoide Arthritis, Lupus-Erkrankungen und andere Kollagenosen, Graves' Disease, Hashimoto's Disease, "Graft-versus-host-Disease" und Transplantatabstossung.

Beispiele für allergische, infektiöse und Toxin-ausgelöste und Ischämieausgelöste Erkrankungen sind: Sarkoidose, Asthma, hypersensitive Pneumonitis, Sepsis, septischer Schock, Endotoxin-Schock, Toxisches Schocksyndrom, Toxisches Leberversagen, ARDS (Akutes Atemnot-Syndrom), Eklampsie, Kachexie, akute Virusinfektionen (z.B. Mononukleose, fulminante Hepatitis), Organschädigung nach Reperfusion.

Ein Beispiel für eine pharmakologisch ausgelöste Entzündung mit pathophysiologischer Relevanz ist die "first dose response" nach Gabe von Anti-T-Zellantikörpern wie OKT3.

Ein Beispiel für systemische Entzündungsreaktionen derzeit unklarer Genese ist die Eklampsie.

Beispiele für neuroinflammatorische Erkrankungen, die mit einer Mikroglia-Aktivierung assoziiert sind, sind AIDS-Dementia, Amyotrophe Lateralsklerose, Creuzfeld-Jacob-Krankheit, Down's Syndrome, diffuse Lewy Body Krankheit, Huntington's Krankheit, Leukencephalopathy, Multiple Sklerose, Parkinsonsche Krankheit, Picksche Krankheit, Alzheimersche Krankheit, Schlaganfall, temporäre Lobe-Epilepsie und Tumore. Daher betrifft die Erfindung auch die Verwendung der angegebenen Benzimidazolderivate zur Behandlung dieser Erkrankungen sowie zur Prophylaxe gegen diese Erkrankungen.

Erfindungsgemäß geeignete Mikroglia-Inhibitoren sind Verbindungen, die bei Stimulierung mit dem Aß-Peptid eine Hemmung der Mikroglia-Aktivität von mindestens 20 % und eine Hemmung der Cytokin-Aktivität von mindestens 30 % erzielen. Die biologischen Eigenschaften der Mikroglia-Inhibitoren können nach den dem Fachmann bekannten Methoden, beispielsweise mit Hilfe der nachfolgend und den in WO 01/51473 beschriebenen Untersuchungsmethoden, gezeigt werden.

In Beispiel 29 ist beschrieben, wie die Hemmung der Mikroglia-Aktivierung gemessen werden kann. Die Mikroglia können dabei durch verschiedene Stimuli aktiviert werden, wie beispielsweise mit Aß-Peptid [β-Amyloid, Araujo, D.M. and Cotman, C.M., Brain Res., 569, 141-145 (1992)], mit Prion-Protein, Zytokinen oder durch Zellfragmente [Combs, C.K. et al., J. Neurosci., 19, 928-939, (1999), Wood, P.L., Neuroinflammation: Mechanisms and Management , Humana Press, (1998)].

Die Stimulierung mit dem Aß-Peptid entspricht der pathophysiologischen Situation bei der Alzheimerschen Krankheit. In diesem Test zeigten die erfindungsgemäßen Substanzen bei Stimulierung mit dem Aß-Peptid eine Hemmung der Mikroglia-Aktvierung. Die Hemmung der Mikroglia-Aktivierung durch die erfindungsgemäßen Substanzen führt zu einer starken Reduktion der Cytokinproduktion und -sekretion, beispielsweise von II1β und TNFα (gemessen durch ELISA und mRNA-Expressionsanalyse), und zu einer verminderten Sekretion von reaktiven Sauerstoff/Stickstoff-Intermediaten. Es werden also gleich mehrere Entzündungsfaktoren gehemmt.

Die *in vivo* Wirksamkeit der erfindungsgemäßen Substanzen wurde in einem MCAO-Modell in Ratten gezeigt. Dieses Modell simuliert den Zustand eines Schlaganfalls. Die erfindungsgemäßen Substanzen reduzieren die Mikroglia-Aktivierung, die bei akuten Hirnlesionen in den Gehirnen der Tiere auftritt.

Die Inhibition der Zytokin-Produktion wird beispielsweise durch Messung von TNFα und Interleukin 12 in Lipopolysaccharid (LPS) stimulierten THP-1 Zellen dargestellt.

Die erfindungsgemäßen Verbindungen inhibieren die TNFα und Interleukin 12 Produktion in Lipopolysanharid (LPS) stimulierten THP-1 Zellen. Zur Darstellung des Einflußes der Substanzen auf die T-Zell-Aktivierung wird beispielsweise die Messung der INFγ Sekretion eingesetzt. Die erfindungsgemäßen Verbindungen inhibieren die INFγ Produktion von peripheren mononukleären Blutzellen.

Ferner betrifft die Erfindung pharmazeutische Mittel, die eine oder mehrere erfindungsgemäße Verbindungen der allgemeinen Formel I sowie einen oder mehrere Trägerstoffe enthalten. Die pharmazeutischen Mittel bzw. Zusammensetzungen der Erfindung werden mit üblichen festen oder flüssigen Trägerstoffen oder Verdünnungsmitteln und üblichen pharmazeutischen und technischen Hilfsstoffen entsprechend der gewünschten Applikationsart mit einer geeigneten Dosierung in an sich bekannter Weise hergestellt. Bevorzugte Zubereitungen bestehen in einer Darreichungsform, die zur oralen, enteralen oder parenteralen, beispielsweise *i.p.* (intraperitonealen), *i.v.* (intravenösen), *i.m.* (intramuskulären), oder perkutanen, Applikation geeignet ist. Solche Darreichungsformen sind beispielsweise Tabletten, Filmtabletten, Dragees, Pillen, Kapseln, Pulver, Cremes, Salben, Lotionen, Flüssigkeiten, wie Sirupe, Gele, injzierbare Flüssigkeiten, beispielsweise zur *i.p.*, *i.v.*, *i.m.*, oder perkutanen Injektion usw. Weiterhin sind auch Depotformen, wie implantierbare Zubereitungen, sowie Suppositorien geeignet. Dabei geben die einzelnen Zubereitungen die erfindungsgemäßen Derivate je nach deren Art allmählich oder die gesamte Menge in kurzer Zeit an den Körper ab.

Zur oralen Verabreichung können Kapseln, Pillen, Tabletten, Dragees und Flüssigkeiten oder andere bekannte orale Darreichungsformen als pharmazeutische Präparate eingesetzt werden. In diesem Falle können die Arzneimittel in der Weise formuliert sein, dass sie die Wirkstoffe entweder in kurzer Zeit freisetzen und an den Körper abgeben oder eine Depotwirkung aufweisen, so dass eine länger anhaltende, langsame Zufuhr von Wirkstoff zum Körper erreicht wird. Die Dosierungseinheiten können neben dem mindestens einen Benzimidazolderivat einen oder mehrere pharmazeutisch verträgliche Träger enthalten, beispielsweise Stoffe zur Einstellung der Rheologie des Arzneimittels, oberflächenaktive Stoffe, Lösungsvermittler, Mikrokapseln, Mikropartikel, Granulate, Verdünner, Bindemittel, wie Stärke, Zucker, Sorbit und Gelatine, ferner Füllstoffe, wie Kieselsäure und Talkum, Gleitmittel, Farbstoffe, Duftstoffe und andere Stoffe.

Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog zu den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Die erfindungsgemäßen Benzimidazolderivate können auch in Form einer Lösung formuliert werden, die für die orale Verabreichung bestimmt ist und die neben dem aktiven Benzimidazolderivat als Bestandteile ein pharmazeutisch verträgliches Öl und/oder eine pharmazeutisch verträgliche lipophile, oberflächenaktive Substanz und/oder eine pharmazeutisch verträgliche hydrophile, oberflächenaktive Substanz und/oder ein pharmazeutisch verträgliches wassermischbares Lösungsmittel enthält.

Um eine bessere Bioverfügbarkeit der erfindungsgemäßen Wirkstoffe zu erreichen, können die Verbindungen auch als Cyclodextrinchlatrate formuliert werden. Hierzu werden die Verbindungen mit α-, β- oder γ-Cyclodextrin oder deren Derivaten umgesetzt.

Falls Cremes, Salben, Lotionen und äusserlich anwendbare Flüssigkeiten eingesetzt werden sollen, müssen diese so beschaffen sein, dass die erfindungsgemäßen Verbindungen dem Körper in ausreichender Menge zugeführt werden. In diesen Darreichungsformen sind Hilfsstoffe enthalten, beispielsweise Stoffe zur Einstellung der Rheologie der Arzneimittel, oberflächenaktive Mittel, Konservierungsmittel, Lösungsvermittler, Verdünner, Stoffe zur Erhöhung der Permeationsfähigkeit für die erfindungsgemäßen Benzimidazolderivate durch die Haut, Farbstoffe, Duftstoffe und Hautschutzmittel, wie Konditionierer und Feuchteregulatoren. Zusammen mit den erfindungsgemäßen Verbindungen können auch andere Wirkstoffe in dem Arzneimittel enthalten sein [Ullmanns Enzyklopädie der technischen Chemie, Band 4 (1953), Seiten 1 - 39; J. Pharm. Sci., 52, 918 ff. (1963); H. v.Czetsch-Lindenwald, Hilfsstoffe für Pharmazie und angrenzende Gebiete; Pharm. Ind., 2, 72 ff (1961); Dr. H. P. Fiedler, Lexikon der Hilfsstoffe für Pharmazie, Kosmetik und angrenzende Gebiete, Cantor AG, Aulendorf/Württ., 1971].

Die erfindungsgemäßen Substanzen können auch in geeigneten Lösungen wie beispielsweise physiologischer Kochsalzlösung, als Infusions- oder Injektionslösung zur Anwendung kommen. Für die parenterale Applikation können die Wirkstoffe in einem physiologisch verträglichen Verdünnungsmittel gelöst oder suspendiert sein. Als Verdünnungsmittel sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden.

Zur Formulierung eines injizierbaren Präparats kann ein beliebiger flüssiger Träger verwendet werden, in dem die erfindungsgemäßen Verbindungen gelöst oder emulgiert sind. Diese Flüssigkeiten enthalten häufig auch Stoffe zur Regulation der Viskosität, oberflächenaktive Stoffe, Konservierungsstoffe, Lösungsvermittler, Verdünner und weitere Zusatzstoffe, mit denen die Lösung isotonisch eingestellt wird. Zusammen mit den Benzimidazolderivaten können auch andere Wirkstoffe verabreicht werden.

Es ist auch möglich, die erfindungsgemäßen Substanzen in ein transdermales System einzuarbeiten und sie damit transdermal zu applizieren. Hierzu werden die Benzimidazolderivate in Form einer Depotinjektion oder eines Implantatpräparats, beispielsweise subkutan, angewendet. Derartige Präparate können so formuliert sein, dass eine verzögerte Wirkstoff-Freigabe ermöglicht wird. Hierzu können bekannte Techniken eingesetzt werden, beispielsweise sich auflösende oder mit einer Membran arbeitende Depots. Implantate können als inerte Materialien beispielsweise biologisch abbaubare Polymere oder synthetische Silikone, beispielsweise Silikonkautschuk, enthalten. Die Benzimidazolderivate können ferner zur perkutanen Verabreichung beispielsweise in ein Pflaster eingearbeitet werden.

Die Dosierung der erfindungsgemäßen Substanzen der allgemeinen Formel I wird vom behandelnden Arzt bestimmt und hängt unter anderem von der verabreichten Substanz, dem Verabreichungsweg, der zu behandelnden Erkrankung und von der Schwere der Erkrankung ab. Die tägliche Dosis beträgt nicht mehr als 1000 mg, vorzugsweise nicht mehr als 100 mg, wobei die Dosis als einmal zu verabreichende Einzeldosis oder unterteilt in zwei oder mehrere Tagesdosen gegeben werden kann.

Die Benzimidazole der Formel I sind auf unterschiedlichen Wegen nach an sich literaturbekannten Verfahren zugänglich.

Als mögliche Verfahren neben anderen seien die folgenden erwähnt:
1. Durch Reaktion von ortho-abgangsgruppen-substituierten (bevorzugt halogensubstituierten) Nitrobenzolderivaten ***(A)*** mit Arylaminen ***(B)*** können *N-*Aryl-2-nitrobenzole ***(C)*** unter diversen Reaktionsbedingungen erzeugt werden, wie zum Beispiel durch Erwärmen der Reaktanden ohne oder mit einem geeigneten inerten Lösungsmittel wie z. B. Alkylbenzolen. Auch kann das als Reaktand verwendete Amin im Überschuss als Lösungsmittel eingesetzt werden. Die Umsetzungen werden sowohl ohne als auch mit Basen (beispielsweise Kaliumcarbonat, Natriumhydrid) durchgeführt. Auch können weitere Hilfsstoffe wie z. B. Kupfersalze Verwendung finden. Beispiele für die hier angegebene Vorgehensweise finden sich in zahlreichen Arbeiten wie etwa in: D. Jerchel, H. Fischer, M. Graft, Ann. Chem., 575,162 (1952) CAS, 53 (2138); R-A. Abramovitch, Can. J. Chem., 38, 2273, 1960). 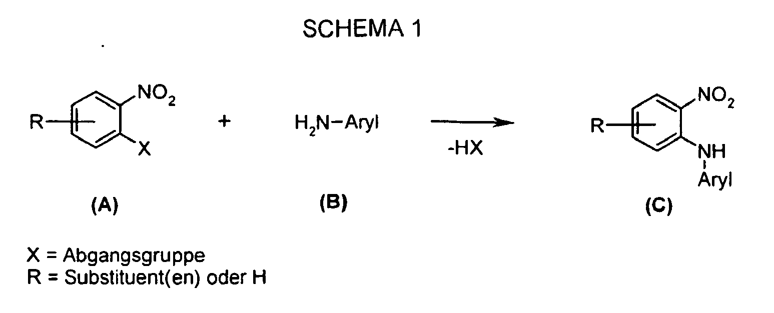 Die so erhaltenen *N-*Arylnitroanilinderivate ***(C)*** lassen sich auf verschiedenen Wegen in 1,2-disubstituierte Benzimidazole ***(E)*** überführen: 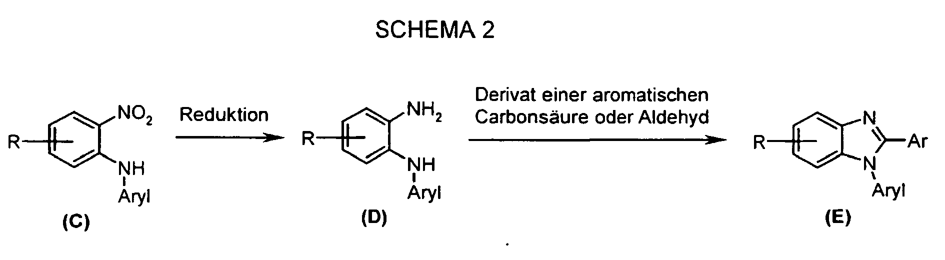 Die Reduktion der Nitrogruppe ***(C)*** wird bevorzugt durch Hydrierung in polaren Lösungsmitteln wie Essigsäure, niederen Alkoholen oder Essigestern unter Zugabe von Katalysatoren, wie Raney-Nickel oder Palladium auf Kohle, oder durch chemische Reduktion beispielsweise mit Zinn in Salzsäure, SnCl₂ [F.D. Bellamy, Tet. Lett., (1984)] oder Fe/Essigsäure [D.C. Owslly, J.J. Bloomfield, Synthesis, 118, 150 (1977)] durchgeführt.
   Aus den Diaminen ***(D)*** sind durch Umsetzung mit Säurederivaten wie Orthoestern, Iminoestern, Säureanhydriden, Aldehyden oder auch freien Carbonsäuren mit oder ohne saure Katalyse und/oder wasserentziehenden Mitteln die Benzimidazole ***(E)*** erhältlich. Als Beispiel sei hier die Darstellung von 1,2-Diphenylbenzimidazol aus Benzoesäure und *N*-Phenyl-o-phenylendiamin unter Verwendung von Triphenylphosphinoxid und Trifluormethansulfonsäureanhydrid angeführt [J. B. Hendrickson, M.S. Hussoin, J. Org. Chem., 52, 4137 (1987)].

   Bei Verwendung von aromatischen Aldehyden wird beispielsweise Nitrobenzol als Lösungsmittel eingesetzt, um die Oxidation des primär gebildeten Benzimidazolins in situ durchführen zu können. Auch gelingt die Cyclisierung zu Benzimidazolen, indem man aromatische Aldehyde als Bisulfitaddukte mit Diaminen ***(D)*** umsetzt.
2. Einen weiteren Zugang zu den Benzimidazolen ***(E)*** beschreiben T. Benincori und F. Sannicolo in J. Heterocyclic Chem. 25, 1029 (1988), der eine breite Variation des Substitutionsmusters sowohl der beiden Arylsubstituenten als auch am Benzolring des Benzimidazoles ermöglicht. Für den Fachmann ist es selbstverständlich, daß diese Substituenten mit den im Verlauf der Synthesesequenz verwendeten Reagenzien und Reaktionsbedingungen verträglich sein müssen. Gelegentlich können die Substituenten später modifiziert werden. Immer ist hier im Produkt eine Hydroxyfunktion in 6-Position des Benzimidazoles enthalten (vgl. Struktur ***F***). 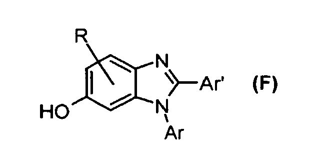
3. Letztlich sei erwähnt, daß in einigen Fällen die Möglichkeit der direkten N-Arylierung von vorgefertigten Benzimidazolen besteht z. B. nach M.J. Sansone, M.S. Kwiatek, US Pat. 4 933 397, oder D.M.T. Chan, K.L. Monaco, R.-P. Wang, M. P. Winters, Tet. Lett. 39 (1998) 2933 oder A.P. Combs, S. Saubern, M. Rafalski, P.Y.S. Lam, Tet. Lett. 40 (1999) 1623.

Für den Fachmann ist es selbstverständlich, dass die Substituenten ***R*** mit den im Verlauf der Synthesesequenz verwendeten Reagenzien und Reaktionsbedingungen verträglich sein müssen. Gegebenenfalls können die Substituenten später modifiziert werden.

Wird das Strukturelement ***B-A-O*** (Formel I) in geschützter oder ungeschützter Form wegen Unverträglichkeit mit den Reaktionsbedingungen während der jeweiligen Benzimidazolsynthese oder aus sonstigen synthetischen Gründen erst nach abgeschlossener Benzimidazolsynthese etabliert, so sind je nach mitgebrachten Substituenten ***R³*** am Benzolring des Benzimidazoles verschiedene Vorgehensweisen zur Etablierung des ***B-A-O-***Strukturelementes (Formel I) möglich, wobei, was dem Fachmann selbstverständlich ist, eine Verträglichkeit der verwendeten Methoden mit den Arylsubstituenten und weiteren Resten ***R³*** berücksichtigt werden muss.

Im folgenden sind einige Möglichkeiten zur Etablierung des ***B-A-O-***Strukturelements aufgezeigt:
Sauerstoff kann in freier Form (z. B. ***R*** = OH in Formel ***(A)***) oder auch in geschützter Form, beispielsweise als Alkylether [vgl. beispielsweise B.D. Jerchel, H. Fischer, M. Graft, Ann. Chem., 575,162 (1952)] von vornherein als Substituent in eine Benzimidazolsynthese mitgebracht werden. Durch Alkyletherspaltung mit z. B. konzentrierter Bromwasserstoffsäure unter eventueller Zuhilfenahme von Lösungsvermittlern wie halogenierten Kohlenwasserstoffen oder auch mit Bortribromid in inerten Lösungsmitteln wie etwa Dichlormethan lässt sich die Hydroxylgruppe freisetzen. Die Hydroxylfunktion lässt sich nach bekannten Methoden mit gegebenenfalls eine endständige Gruppe ***B*** (Formel I), oder eine Vorstufe davon enthaltenden Alkylhalogeniden zu den Ethern umsetzen, wobei die Umsetzung mit den Alkylierungsmitteln bevorzugt in polaren Lösungsmitteln wie etwa Dimethylformamid, Dimethylsulfoxid, Ethern, wie etwa Tetrahydrofuran oder auch niederen Ketonen, wie Aceton oder Methylethylketon, unter Zugabe von Basen, wie Alkali- und Erdalkalihydriden, bevorzugt jedoch Natriumhydrid, oder unter Zugabe von Alkalicarbonaten, wie Kalium- oder Cäsiumcarbonat, durchgeführt wird in einem Temperaturbereich von 0°C bis 120°C. Des Weiteren kann eine Umsetzung in einem Zweiphasensystem unter Phasentransferkatalyse erfolgen, wobei die Reaktanden in einem geeigneten inerten organischen Lösungsmittel gelöst werden wie beispielsweise in Halogenalkanen, bevorzugt jedoch in Dichlormethan. Die andere Phase ist ein festes Alkalihydroxid, bevorzugt Natrium- oder Kaliumhydroxid, oder auch eine konzentrierte wässrige Lösung des betreffenden Hydroxids. Als Phasentransferkatalysatoren werden beispielsweise quartäre Ammoniumsalze verwendet. Reaktionen unter Phasentransferkatalyse werden bevorzugt bei Raumtemperatur durchgeführt.

Beispielsweise wird eine Verbindung der Formel ***A*** (mit R = OH) in Dimethylformamid gelöst und unter Zugabe von Cäsiumcarbonat mit 6-Bromhexansäuremethylester bei Temperaturen von 0°C bis 50°C umgesetzt. Die Spaltung des Esters durch saure oder alkalische Hydrolyse lässt sich nach den dem Fachmann bekannten Methoden durchführen, wie beispielsweise mit basischen Katalysatoren wie beispielsweise mit Alkali- oder Erdalkalicarbonaten oder -hydroxiden in Wasser oder der wässrigen Lösung eines Alkohols. Als Alkohole kommen aliphatische Alkohole wie beispielsweise Methanol, Ethanol, Butanol etc. in Betracht, vorzugsweise jedoch Methanol. Auch wässrige Lösungen von Ethern wie Tetrahydrofuran finden Verwendung. Als Alkalicarbonate und -hydroxide seien Lithium-, Natrium- und Kaliumsalze genannt. Bevorzugt sind die Lithium- und Natriumsalze. Als Erdalkalicarbonate und -hydroxide eignen sich beispielsweise Calciumcarbonat, Calciumhydroxid und Bariumcarbonat. Die Umsetzung erfolgt allgemein bei -10°C bis 70°C, vorzugsweise jedoch bei 25°C. Die Esterspaltung kann jedoch auch unter sauren Bedingungen, wie etwa in wässriger Salzsäure, gegebenenfalls unter Zuhilfenahme eines Lösungsvermittlers, wie etwa einem niederen Alkohol, bevorzugt Methanol, erfolgen.

Aus einem im Alkylierungsreagenz vorhandenen oder auch nachträglich generierten Nitril lässt sich durch Hydrolyse eine Carbonsäurefunktion generieren. Die Alkylierungsreagenzien können auch funktionelle Gruppen wie etwa Hydroxylfunktionen in freier oder geschützter Form enthalten, welche sich nach Überführung in Abgangsgruppen, wie beispielsweise Tosylat, Mesylat, Bromid oder Jodid, beispielsweise gegen Amino-, oder Alkylgruppen austauschen lassen. Auch können die Alkylierungsreagenzien funktionelle Gruppen, wie beispielsweise Halogene oder gegebenenfalls geschützte Aminogruppen, enthalten, die dann weiter modifiziert werden können.

Je nach angestrebter Substitution sind die Substituenten ***R³*** von vornherein in den Synthesebausteinen enthalten oder werden nach Bedarf an geeigneter Stelle der betreffenden Synthesesequenz etabliert beziehungsweise aus mitgebrachten geeigneten Vorläufern generiert.

Die freien Säurederivate der Formel I oder Estervorstufen können nach diversen, literaturbekannten Verfahren in Amidderivate der Formel I überführt werden.

Die freien Säurederivate der Formel I lassen sich auch mit geeigneten Mengen der entsprechenden anorganischen Basen unter Neutralisierung in Salze überführen. Beispielsweise erhält man beim Lösen der entsprechenden Säuren in Wasser, welches stöchiometrische Mengen der Base enthält, nach Abdampfen des Wassers oder nach Zugabe eines mit Wasser mischbaren Lösungsmittels, zum Beispiel Alkohol oder Aceton, das feste Salz.

Die Aminsalze können in üblicher Weise hergestellt werden. Dazu löst man die entsprechende Säure in einem geeigneten Lösungsmittel, wie beispielsweise Ethanol, Aceton, Diethylether oder Benzol, und setzt ein bis fünf Äquivalente des jeweiligen Amins dieser Lösung zu. Dabei fällt das Salz gewöhnlich in fester Form an oder wird nach dem Verdampfen des Lösungsmittels in üblicher Weise isoliert.

Die Clathrate mit α-, β- oder γ-Cyclodextrin werden analog der Vorschrift in WO-A-87/05294 erhalten. Bevorzugt wird β-Cyclodextrin verwendet. Liposomen werden nach dem in Pharmazie in unserer Zeit, 11, 98 (1982) beschriebenen Verfahren hergestellt.

Nachfolgend wird die Herstellung einiger Vorstufen, Zwischenprodukte und Produkte exemplarisch beschrieben. Soweit die Herstellung der Ausgangsverbindungen nicht beschrieben wird, sind die Ausgangsverbindungen bekannt und käuflich, oder die Verbindungen werden analog zu den beschriebenen Verfahren synthetisiert.

Bei der Herstellung der erfindungsgemäßen Substanzen bedient man sich beispielsweise folgender Verfahren:

### Allgemeine Arbeitsvorschrift 1:

### Reduktion Nitrogruppen

Die zu reduzierende Verbindung wird in Ethylacetat, Tetrahydrofuran, Methanol oder Ethanol oder Gemischen der Lösungsmittel gelöst und an 2-5% (bezogen auf die Nitroverbindung) Palladium auf Kohle (10%) bei normalem Druck hydriert. Nach Ende der Wasserstoffaufnahme wird abgesaugt, der Rückstand mit Ethylacetat oder Methanol oder Ethanol gewaschen und das Filtrat im Vakuum eingeengt. Das Rohprodukt wird in der Regel ohne weitere Reinigung umgesetzt.

### Allgemeine Arbeitsvorschrift 2:

### Etherspaltung mit Bromwasserstoffsäure

5 g Arylmethylether werden mit 160 ml 48%iger wäßriger HBr versetzt und für 1-5 h zum Rückfluß erhitzt. Nach dem Erkalten wird filtriert. Der Rückstand wird in Ethylacetat aufgenommen, und dreimal mit ges. Natriumhydrogencarbonatlösung extrahiert. Nach Trocknung über über Natriumsulfat wird im Vakuum eingeengt. Der Rückstand wird falls erforderlich durch Kristallisation oder Säulenchromatographie an Kieselgel gereinigt.

### Allgemeine Arbeitsvorschrift 3:

### Alkylierung von Hydroxybenzimidazolderivaten und Phenolderivaten mit Alkylhalogeniden

Eine Lösung von 1.85 mmol des Hydroxybenzimidazolderivats in 12 ml *N*,*N-*Dimethylformamid wird mit 1.85 mmol Caesiumcarbonat, und 2.24 mmol Alkylbromid oder Alkyljodid versetzt. Bei Verwendung der Alkylbromide werden optional 1.85 mmol Natriumjodid zugesetzt. Man rührt für 12-96 h, gießt dann auf Wasser, nimmt mit Ethylacetat auf, wäscht die organische Phase viermal mit Wasser, trocknet die über Natriumsulfat und engt im Vakuum ein.
Alternativ zu dieser wäßrigen Aufarbeitung kann man das Reaktionsgemisch mit Dichlormethan versetzen, von den ausfallenden Salzen durch Filtration trennen und das Filtrat im Vakuum einengen.
Unabhängig von der Aufarbeitungsmethode wird der Rückstand durch Kristallisation oder Säulenchromatographie an Kieselgel gereinigt.

### Allgemeine Arbeitsvorschrift 4:

### Verseifung von Carbonsäurealkylestern

0.77 mmol des Carbonsäurealkylesters werden in 5 ml Methanol und 5 ml Tetrahydrofuran gelöst und mit 5 ml einer 0.5 N wäßrigen Lithium- oder Natriumhydroxidlösung versetzt. Nach 2-12 h Rühren wird im Vakuum weitestgehend eingeengt, durch Zusatz von wäßriger Salzsäure neutralisiert und mit Ethylacetat extrahiert. Man trocknet über Natriumsulfat und engt im Vakuum ein. Der Rückstand wird falls erforderlich durch Säulenchromatographie an Kieselgel gereinigt.

### Allgemeine Arbeitsvorschrift 5:

### Cyclisierung zu Benzimidazolen mit Aldehyden

1 mmol eines 1,2-Diaminobenzolderivats werden in 3 ml Nitrobenzol gelöst. Dazu gibt man 1 mmol eines Aryl- bzw. Heteroarylaldehyds. Man erhitzt für 2-6 h auf 150 °C und lässt erkalten. Der Rückstand wird ohne weitere Aufarbeitung direkt durch Säulenchromatographie an Kieselgel gereinigt.

### Allgemeine Arbeitsvorschrift 6:

### Überführung von Carbonsäureestern in Carbonsäureamide

0.36 mmol eines Amins werden in 3 ml Toluol gelöst und unter Kühlung im Eisbad tropfenweise mit 0.18 ml einer 2 M Lösung von Trimethyaluminium in Toluol versetzt. Man versetzt mit einer Lösung aus 0.33 mmol des Carbonsäuremethylesters in 3 ml Toluol und rührt 2-8 h bei 95°C. Zur Aufarbeitung gibt man nach dem Erkalten Wasser zu, extrahiert dreimal mit Ethylacetat, wäscht die vereinigten organischen Phasen mit ges. Natriumchloridlösung, trocknet über Natriumsulfat und engt im Vakuum ein. Der Rückstand wird durch Säulenchromatographie an Kieselgel gereinigt.

### Vergleichs-Beispiel 1

### 6-[[1-(4-Methylphenyl)-2-(3-pyridinyl)-1H-benzimidazol-6-yl]oxy]hexansäure

### a) 3-(4-Methylphenyl)amino-4-nitrophenol

5.4 g 3-Fluor-4-nitrophenol und 4.8 ml 4-Methylanilin wurden vermischt und für 6 h bei 120°C gerührt. Nach dem Erkalten wurde in Ethylacetat und Wasser aufgenommen und dreimal mit 1 N wäßriger Salzsäure extrahiert. Die vereinigten wäßrigen Phasen wurden dreimal mit Ethylacetat extrahiert. Die vereinigten organischen Phasen wurden über Natriumsulfat getrocknet, im Vakuum eingeengt und der Rückstand kristallisiert.
MS (EI): 244 (Molekülionpeak)

### b) 6-[3-(4-Methylphenyl)amino-4-nitrophenyl]oxyhexansäuremethylester

wurde durch Umsetzung von 3-(4-Methylphenyl)amino-4-nitrophenol mit 6-Bromhexansäuremethylester gemäß der allgemeinen Arbeitsvorschrift 3 erhalten.
MS (EI): 372 (Molekülionpeak)

### c) 6-[[4-Amino-3-((4-methylphenyl)amino)phenyl]oxy]hexansäuremethylester

wurde durch Umsetzung von 6-[3-(4-Methylphenyl)amino-4-nitrophenyl]oxyhexansäuremethylester gemäß der allgemeinen Arbeitsvorschrift 1 erhalten.
MS (EI): 342 (Molekülionpeak)

### d) 6-[[1-(4-Methylphenyl)-2-(3-pyridinyl)-1H-benzimidazol-6-yl]oxy]hexansäuremethylester

wurde durch Umsetzung aus 6-[[4-Amino-3-((4-methylphenyl)amino)-phenyl]oxy]hexansäuremethylester mit 3-Pyridylcarbaldehyd gemäß der allgemeinen Arbeitsvorschrift 5 erhalten.
MS (EI): 429 (Molekülionpeak)

### e) 6-[[1-(4-Methylphenyl)-2-(3-pyridinyl)-1H-benzimidazol-6-yl]oxy]hexansäure

wurde durch Umsetzung von 6-[[1-(4-Methylphenyl)-2-(3-pyridinyl)-1H-benzimidazol-6-yl]oxy]hexansäuremethylester gemäß der allgemeinen Arbeitsvorschrift 4 dargestellt.
MS (EI): 415 (Molekülionpeak)

### Beispiel 2

### 5-[[1-(4-Methylphenyl)-2-(3-pyridinyl)-1H-benzimidazol-6-yl]oxy]pentansäure

### a) 5-[3-(4-Methylphenyl)amino-4-nitrophenyl]oxypentansäuremethylester

wurde durch Umsetzung von 3-(4-Methylphenyl)amino-4-nitrophenol mit 5-Brompentansäuremethylester gemäß der allgemeinen Arbeitsvorschrift 3 erhalten.
MS (EI): 358 (Molekülionpeak)

### b) 5-[[4-Amino-3-((4-methylphenyl)amino)phenyl]oxy]pentansäuremethylester

wurde durch Umsetzung von 5-[3-(4-Methylphenyl)amino-4-nitrophenyl]-oxypentansäuremethylester gemäß der allgemeinen Arbeitsvorschrift 1 erhalten.
MS (EI): 328 (Molekülionpeak)

### c) 5-[[1-(4-Methylphenyl)-2-(3-pyridinyl)-1H-benzimidazol-6-yl]oxy]pentansäuremethylester

wurde durch Umsetzung aus 5-[[4-Amino-3-((4-methylphenyl)amino)-phenyl]oxy]pentansäuremethylester mit 3-Pyridylcarbaldehyd gemäß der allgemeinen Arbeitsvorschrift 5 erhalten.
MS (EI): 415 (Molekülionpeak)

### d) 5-[[1-(4-Methylphenyl)-2-(3-pyridinyl)-1H-benzimidazol-6-yl]oxy]pentansäure

wurde durch Umsetzung von 5-[[1-(4-Methylphenyl)-2-(3-pyridinyl)-1*H-*benzimidazol-6-yl]oxy]pentansäuremethylester gemäß der allgemeinen Arbeitsvorschrift 4 dargestellt.
MS (EI): 401 (Molekülionpeak)

### Beispiel 3

### 4-[[1-(4-Methylphenyl)-2-(3-pyridinyl)-1H-benzimidazol-6-yl]oxy]buttersäure

### a) 4-[3-(4-Methylphenyl)amino-4-nitrophenyl]oxybuttersäuremethylester

wurde durch Umsetzung von 3-(4-Methylphenyl)amino-4-nitrophenol mit 4-Brombuttersäuremethylester gemäß der allgemeinen Arbeitsvorschrift 3 erhalten.
MS (EI): 344 (Molekülionpeak)

### b) 4-[[4-Amino-3-((4-methylphenyl)amino)phenyl]oxy]buttersäuremethylester

wurde durch Umsetzung von 4-[3-(4-Methylphenyl)amino-4-nitrophenyl]-oxybuttersäuremethylester gemäß der allgemeinen Arbeitsvorschrift 1 erhalten.
MS (EI): 314 (Molekülionpeak)

### c) 4-[[1-(4-Methylphenyl)-2-(3-pyridinyl)-1H-benzimidazol-6-yl]oxy]buttersäuremethylester

wurde durch Umsetzung von 4-[[4-Amino-3-((4-methylphenyl)amino)-phenyl]oxy]buttersäuremethylester mit 3-Pyridylcarbaldehyd gemäß der allgemeinen Arbeitsvorschrift 5 erhalten.
MS (EI): 401 (Molekülionpeak)

### d) 4-[[1-(4-Methylphenyl)-2-(3-pyridinyl)-1H-benzimidazol-6-yl]oxy]buttersäure

wurde durch Umsetzung von 4-[[1-(4-Methylphenyl)-2-(3-pyridinyl)-1*H-*benzimidazol-6-yl]oxy]buttersäuremethylester gemäß der allgemeinen Arbeitsvorschrift 4 dargestellt.
MS (EI): 387 (Molekülionpeak)

### Vergleichs-Beispiel 4

### 6-[[1-(4-Methylphenyl)-2-(4-pyridinyl)-1H-benzimidazol-6-yl]oxy]hexansäure

### a) 6-[[1-(4-Methylphenyl)-2-(4-pyridinyl)-1H-benzimidazol-6-yl]oxy]hexansäuremethylester

wurde durch Umsetzung aus 6-[[4-Amino-3-((4-methylphenyl)amino)-phenyl]oxy]hexansäuremethylester mit 4-Pyridylcarbaldehyd gemäß der allgemeinen Arbeitsvorschrift 5 erhalten.
MS (EI): 429 (Molekülionpeak)

### b) 6-[[1-(4-Methylphenyl)-2-(4-pyridinyl)-1H-benzimidazol-6-yl]oxy]hexansäure

wurde durch Umsetzung von 6-[[1-(4-Methylphenyl)-2-(4-pyridinyl)-1*H-*benzimidazol-6-yl]oxy]hexansäuremethylester gemäß der allgemeinen Arbeitsvorschrift 4 dargestellt.
MS (EI): 415 (Molekülionpeak)

### Vergleichs-Beispiel 5

### 6-[[1-(4-Methylphenyl)-2-(3-thienyl)-1H-benzimidazol-6-yl]oxy]hexansäure

### a) 6-[[1-(4-Methylphenyl)-2-(3-thienyl)-1H-benzimidazol-6-yl]oxy]hexansäuremethylester

wurde durch Umsetzung von 6-[[4-Amino-3-((4-methylphenyl)amino)-phenyl]oxy]hexansäuremethylester mit 3-Thienylcarbaldehyd gemäß der allgemeinen Arbeitsvorschrift 5 erhalten.
MS (EI): 434 (Molekülionpeak)

### b) 6-[[1-(4-Methylphenyl)-2-(3-thienyl)-1H-benzimidazol-6-yl]oxy]hexansäure

wurde durch Umsetzung von 6-[[1-(4-Methylphenyl)-2-(3-thienyl)-1*H-*benzimidazol-6-yl]oxy]hexansäuremethylester gemäß der allgemeinen Arbeitsvorschrift 4 dargestellt.
MS (EI): 420 (Molekülionpeak)

### Beispiel 6

### 5-[[1-(4-Methylphenyl)-2-(3-thienyl)-1H-benzimidazol-6-yl]oxy]pentansäure

### a) 5-[[1-(4-Methylphenyl)-2-(3-thienyl)-1H-benzimidazol-6-yl]oxy]pentansäuremethylester

wurde durch Umsetzung von 5-[[4-Amino-3-((4-methylphenyl)-amino)phenyl]oxy]pentansäuremethylester mit 3-Thienyl-carbaldehyd gemäß der allgemeinen Arbeitsvorschrift 5 erhalten.
MS (EI): 420 (Molekülionpeak)

### b) 5-[[1-(4-Methylphenyl)-2-(3-thienyl)-1H-benzimidazol-6-yl]oxy]pentansäure

wurde durch Umsetzung von 5-[[1-(4-Methylphenyl)-2-(3-thienyl)-1*H-*benzimidazol-6-yl]oxy]pentansäuremethylester gemäß der allgemeinen Arbeitsvorschrift 4 dargestellt.
MS (EI): 406 (Molekülionpeak)

### Beispiel 7

### 4-[[1-(4-Methylphenyl)-2-(3-thienyl)-1H-benzimidazol-6-yl]oxy]buttersäure

### a) 4-[[1-(4-Methylphenyl)-2-(3-thienyl)-1H-benzimidazol-6-yl]oxy]buttersäuremethylester

wurde durch Umsetzung von 4-[[4-Amino-3-((4-methylphenyl)-amino)phenyl]oxy]buttersäuremethylester mit 3-Thiophencarbaldehyd gemäß der allgemeinen Arbeitsvorschrift 5 erhalten.
MS (EI): 406 (Molekülionpeak)

### b) 4-[[1-(4-Methylphenyl)-2-(3-thienyl)-1H-benzimidazol-6-yl]oxy]buttersäure

wurde durch Umsetzung von 4-[[1-(4-Methylphenyl)-2-(3-thienyl)-1*H-*benzimidazol-6-yl]oxy]buttersäuremethylester gemäß der allgemeinen Arbeitsvorschrift 4 dargestellt.
MS (EI): 392 (Molekülionpeak)

### Beispiel 8

### 5-[[1-Phenyl-2-(3-thienyl)-1H-benzimidazol-6-yl]oxy]pentansäure

### a) 4-Methoxy-N²-phenyl-o-phenylendiamin

wurde durch Umsetzung von (5-Methoxy-2-nitrophenyl)phenylamin [Kottenhahn et al.; J.Org.Chem.; 28; 1963; 3114,3118; Banthorpe; Cooper; J.Chem.Soc.B; 1968; 605] gemäß der allgemeinen Arbeitsvorschrift 1 erhalten.
¹H-NMR (CDCl₃): δ = 3.42 ppm s (breit)(2H); 3.72 s (3H); 5.33 s (breit)(1H); 6.56 dd (J = 10, 2 Hz, 1H); 6.76 d (J = 10 Hz, 1H); 6.79 d (J = 2 Hz, 1H); 6.82-6.90 m (3H); 7.25 dd (J = 8, 8 Hz, 2H).

### b) 6-Methoxy-1-phenyl-2-(3-thienyl)-1H-benzimidazol

wurde durch Umsetzung von 4-Methoxy-*N*²-phenyl-o-phenylendiamin mit Thiophen-3-carbaldehyd gemäß der allgemeinen Arbeitsvorschrift 5 erhalten.
MS (EI): 306 (Molekülionpeak)

### c) 6-Hydroxy-1-phenyl-2-(3-thienyl)-1H-benzimidazol

wurde durch Umsetzung von 6-Methoxy-1-phenyl-2-(3-thienyl)-1*H-*benzimidazol gemäß der allgemeinen Arbeitsvorschrift 2 erhalten.
MS (EI): 292 (Molekülionpeak)

### d) 5-[[1-Phenyl-2-(3-thienyl)-1H-benzimidazol-6-yl]oxy]pentansäuremethylester

wurde durch Umsetzung von 6-Hydroxy-1-phenyl-2-(3-thienyl)-1*H-*benzimidazol mit 5-Brompentansäuremethylester gemäß der allgemeinen Arbeitsvorschrift 3 erhalten.
MS (EI): 406 (Molekülionpeak)

### e) 5-[[1-Phenyl-2-(3-thienyl)-1H-benzimidazol-6-yl]oxy]pentansäure

wurde durch Umsetzung von 5-[[1-Phenyl-2-(3-thienyl)-1*H*-benzimidazol-6-yl]oxy]pentansäuremethylester gemäß der allgemeinen Arbeitsvorschrift 4 erhalten.
MS (EI): 392 (Molekülionpeak)

### Beispiel 9

### 4-[[1-Phenyl-2-(3-thienyl)-1H-benzimidazol-6-yl]oxy]buttersäure

### a) 4-[[1-Phenyl-2-(3-thienyl)-1H-benzimidazol-6-yl]oxy]buttersäuremethylester

wurde durch Umsetzung von 6-Hydroxy-1-phenyl-2-(3-thienyl)-1*H-*benzimidazol mit 4-Brombutansäuremethylester gemäß der allgemeinen Arbeitsvorschrift 3 erhalten.
MS (EI): 392 (Molekülionpeak)

### b) 4-[[1-Phenyl-2-(3-thienyl)-1H-benzimidazol-6-yl]oxy]buttersäure

wurde durch Umsetzung von 4-[[1-Phenyl-2-(3-thienyl)-1*H-*benzimidazol-6-yl]oxy]buttersäuremethylester gemäß der allgemeinen Arbeitsvorschrift 4 erhalten.
MS (EI): 378 (Molekülionpeak)

### Vergleichs-Beispiel 10

### 6-[[1-Phenyl-2-(3-thienyl)-1H-benzimidazol-6-yl]oxy]hexansäure

### a) 6-[[1-Phenyl-2-(3-thienyl)-1H-benzimidazol-6-yl]oxy]hexansäuremethylester

wurde durch Umsetzung von 6-Hydroxy-1-phenyl-2-(3-thienyl)-1*H-*benzimidazol mit 6-Bromhexansäuremethylester gemäß der allgemeinen Arbeitsvorschrift 3 erhalten.
MS (EI): 420 (Molekülionpeak)

### b) 6-[[1-Phenyl-2-(3-thienyl)-1H-benzimidazol-6-yl]oxy]hexansäure

wurde durch Umsetzung von 6-[[1-Phenyl-2-(3-thienyl)-1*H-*benzimidazol-6-yl]oxy]hexansäuremethylester gemäß der allgemeinen Arbeitsvorschrift 4 erhalten.
MS (EI): 406 (Molekülionpeak)

### Vergleichs-Beispiel 11

### 6-[[1-(4-Fluorphenyl)-2-(3-thienyl)-1H-benzimidazol-6-yl]oxy]hexansäure

### a) (5-Chlor-2-nitrophenyl)-(4-fluorophenyl)amin

50 g 1-Chlor-3,4-dinitrobenzol in 250 ml Ethanol werden mit 50 ml 4-Fluoranilin versetzt und 35 Stunden bei 60°C gerührt. Nach Einengen auf das halbe Volumen wird zwischen Wasser und Dichlormethan verteilt. Nach Waschen der organischen Phase mit 1 N wäßriger Salzsäure wird über Natriumsulfat getrocknet, filtriert und eingeengt. Nach Chromatographie an Kieselgel werden 62,33g (5-Chlor-2-nitrophenyl)-(4-fluorophenyl)amin erhalten.
¹H-NMR (CDCl₃): δ = 6.71 dd (J = 9, 2 Hz, 1H); 6.97 d (J = 2 Hz, 1H); 7,13 dd (J = 9, 9 Hz, 2H); 7,22 dd (J = 9, 6 Hz, 2H); 8,15 d (J = 10 Hz, 1H); 9.45 s (br)(1H).

### b) (5-Methoxy-2-nitrophenyl)-(4-fluorphenyl)amin

Zu einer Lösung aus 6,6 g Natrium in 450 ml Methanol gab man 36,44 g (5-Chlor-2-nitrophenyl)-(4-fluorphenyl)amin und erhitzte für 16 h zum Rückfluß. Nach weiteren 30 h Rühren bei 60°C wurde abgekühlt und das kristalline Produkt abgesaugt. Es wurden 34 g (5-Methoxy-2-nitrophenyl)-(4-fluorphenyl)amin erhalten.
¹H-NMR (CDCl₃): δ = 3.72 s (3H); 6.44 dd (J = 9, 2 Hz, 1H); 6,48 d (J = 2 Hz, 1H); 7,13 dd (J = 9, 9 Hz, 2H); 7.27 dd (9, 6 Hz, 2H); 8.20 d (J = 9 Hz, 1H); 9.65 s (br)(1H).

### c) N²-(4-Fluorphenyl)-4-methoxybenzol-1,2-diamin

33,5 g (0,128 ml) (5-Methoxy-2-nitrophenyl)-(4-fluorphenyl)amin wurden gemäß der allgemeinen Arbeitsvorschrift 1 umgesetzt. Das Rohprodukt wurde ohne Reinigung weiterverarbeitet.
¹H-NMR (CDCl₃): δ = 3,70 ppm s (3H); 6.49 d(br) (J = 9 Hz, 1H); 6,68 d (J = 2 Hz, 1H); 6,78-6,97 m (5H).

### d) 6-Methoxy-1-(4-fluorphenyl)-2-(3-thienyl)-1H-benzimidazol

7,48 g Thiophen-3-aldehyd wurden in 65 ml 40%-iger NaHSO₃-Lösung zwei Stunden gerührt. Nach Zugabe von 15 g *N*²-(4-Fluorphenyl)-4-methoxybenzol-1,2-diamin in 50 ml Ethanol wurde 4 Stunden gekocht und über Nacht nachgerührt. Der Ansatz wurde zwischen Wasser und Ethylacetat verteilt und die organische Phase mit Wasser gewaschen. Nach Trocknung über Natriumsulfat und Einengen des Filtrats werden 18,1 g rohes 6-Methoxy-1-(4-fluorphenyl)-2-(3-thienyl)-1*H-*benzimidazol erhalten.
Fp. 154-158°C

### e) 6-Hydroxy-1-(4-fluorphenyl)-2-(3-thienyl)-1H-benzimidazol

18 g (55,5 mmol) 6-Methoxy-1-(4-fluorphenyl)-2-(3-thienyl)-1*H-*benzimidazol werden analog der allgemeinen Arbeitsvorschrift 2 umgesetzt. Es wurden 12,65 g (40 mmol) rohes 6-Hydroxy-1-(4-fluorphenyl)-2-(3-thienyl)-1*H-*benzimidazol erhalten.
Fp. 212-218°C

### f) 6-[[1-(4-Fluorphenyl)-2-(3-thienyl)-1H-benzimidazol-6-yl]oxy]hexansäuremethylester

6-Hydroxy-1-(4-fluorphenyl)-2-(3-thienyl)-1*H-*benzimidazol wurde mit 6-Bromhexansäuremethylester gemäß der allgemeinen Arbeitsvorschrift 3 umgesetzt.
Fp. 131-134°C

### g) 6-[[1-(4-Fluorphenyl)-2-(3-thienyl)-1H-benzimidazol-6-yl]oxy]hexansäure

wurde durch Umsetzung von 6-[[1-(4-Fluorphenyl)-2-(3-thienyl)-1*H-*benzimidazol-6-yl]oxy]hexansäuremethylester gemäß der allgemeinen Arbeitsvorschrift 4 erhalten.
Fp. 170-175°C

### Beispiel 12

### 5-[[1-(4-Fluorphenyl)-2-(3-thienyl)-1H-benzimidazol-6-yl]oxy]pentansäure

### a) 5-[[1-(4-Fluorphenyl-2-(3-thienyl)-1H-benzimidazol-6-yl]oxy]pentansäuremethylester

6-Hydroxy-1-(4-fluorphenyl)-2-(3-thienyl)-1*H-*benzimidazol wurde gemäß der allgemeinen Arbeitsvorschrift 3 mit 5-Brompentansäuremethylester umgesetzt.
Fp. 90,5-92,5°C

### b) 5-[[1-(4-Fluorphenyl)-2-(3-thienyl)-1H-benzimidazol-6-yl]oxy]pentansäure

5-[[1-(4-Fluorphenyl-2-(3-thienyl)-1*H*-benzimidazol-6-yl]oxy]pentansäuremethylester wurde gemäß der allgemeinen Vorschrift 4 umgesetzt.
Fp. 184-189°C

### Vergileichs-Beispiel 13

### 6-[[1-(4-Fluorphenyl)-2-(3-pyridinyl)-1H-benzimidazol-6-yl]oxy]hexansäure

### a) 6-Methoxy-1-(4-fluorphenyl)-2-(3-pyridinyl)-1H-benzimidazol

*N*²-(4-Fluorphenyl)-4-methoxybenzol-1,2-diamin wurde analog Beispiel 11d mit Pyridin-3-carbaldehyd umgesetzt.
Fp. 132,5-134°C

### b) 6-Hydroxy-1-(4-fluorphenyl)-2-(3-pyridinyl)-1H-benzimidazol

6-Methoxy-1-(4-fluorphenyl)-2-(3-pyridinyl)-1*H-*benzimidazol wurde gemäß der allgemeinen Arbeitsvorschrift 2 umgesetzt.
Fp. 238-241°C

### c) 6-[[1-(4-Fluorphenyl)-2-(3-pyridinyl)-1H-benzimidazol-6-yl]oxy]hexansäuremethylester

6-Hydroxy-1-(4-fluorphenyl)-2-(3-pyridinyl)-1*H-*benzimidazol wurde mit 6-Bromhexansäuremethylester gemäß der allgemeinen Arbeitsvorschrift 3 umgesetzt.
Fp. 105.5-111,5°C

### d) 6-[[1-(4-Fluorphenyl)-2-(3-pyridinyl)-1H-benzimidazol-6-yl]oxy]hexansäure

wurde durch Umsetzung von 6-[[1-(4-Fluorphenyl)-2-(3-pyridinyl)-1*H-*benzimidazol-6-yl]oxy]hexansäuremethylester gemäß der allgemeinen Arbeitsvorschrift 4 erhalten.
Fp. 127,5-129°C

### Beispiel 14

### 5-[[1-(4-Fluorphenyl)-2-(3-pyridinyl)-1H-benzimidazol-6-yl]oxy]pentansäure

### a) 5-[[1-(4-Fluorphenyl)-2-(3-pyridinyl)-1H-benzimidazol-6-yl]oxy]pentansäuremethylester

6-Hydroxy-1-(4-fluorphenyl)-2-(3-pyridinyl)-1*H-*benzimidazol wurde mit 5-Brompentansäuremethylester gemäß der allgemeinen Arbeitsvorschrift 3 umgesetzt.
Fp. 52-55°C

### b) 5-[[1-(4-Fluorphenyl)-2-(3-pyridinyl)-1H-benzimidazol-6-yl]oxy]pentansäure

wurde durch Umsetzung von 5-[[1-(4-Fluorphenyl)-2-(3-pyridinyl)-1*H-*benzimidazol-6-yl]oxy]pentansäuremethylester gemäß der allgemeinen Arbeitsvorschrift 4 erhalten.
Fp. 181,5-183°C

### Beispiel 15

### 5-[[1-Phenyl-2-(3-pyridinyl)-1H-benzimidazol-6-yl]oxy]pentansäure

### a) 6-Methoxy-1-phenyl-2-(3-pyridinyl)-1H-benzimidazol

wurde durch Umsetzung von 4-Methoxy-*N*²-phenyl-*o*-phenylendiamin mit Pyridin-3-carbaldehyd gemäß der allgemeinen Arbeitsvorschrift 5 erhalten.
MS (EI): 301 (Molekülionpeak)

### b) 6-Hydroxy-1-phenyl-2-(3- pyridinyl)-1H-benzimidazol

wurde durch Umsetzung von 6-Methoxy-1-phenyl-2-(3-pyridinyl)-1*H-*benzimidazol gemäß der allgemeinen Arbeitsvorschrift 2 erhalten.
¹H-NMR (D₆-DMSO): δ = 6.52 ppm d (J = 2 Hz, 1H); 6.81 dd (J = 8, 2 Hz, 1H); 7.34-7.48 m (3H); 7.53-7.68 m (4H); 7.80 (ddd, J = 8, 2, 1Hz, 1H); 8.53 dd (J = 2, 1Hz, 1H); 8.67 d (J = 1Hz, 1H); 9.42 (s, 1H).

### c) 5-[[1-Phenyl-2-(3-pyridinyl)-1H-benzimidazol-6-yl]oxy]pentansäuremethylester

wurde durch Umsetzung von 6-Hydroxy-1-phenyl-2-(3-pyridinyl)-1*H-*benzimidazol mit 5-Brompentansäuremethylester gemäß der allgemeinen Arbeitsvorschrift 3 erhalten.
MS (EI): 401 (Molekülionpeak)

### d) 5-[[1-Phenyl-2-(3-pyridinyl)-1H-benzimidazol-6-yl]oxy]pentansäure

wurde durch Umsetzung von 5-[[1-Phenyl-2-(3-pyridinyl)-1*H-*benzimidazol-6-yl]oxy]pentansäuremethylester gemäß der allgemeinen Arbeitsvorschrift 4 erhalten.
¹H-NMR (CD₃OD): δ = 1.72-1.88 m (4H); 2.30 t (J = 8 Hz, 2H); 3.98 t (J = 8 Hz, 2H); 6.72 ppm d (J = 2 Hz, 1H); 7.03 dd (J = 8, 2 Hz, 1H); 7.40-7.48 m (3H); 7.55-7.65 m (3H); 7.70 (d, J = 8 Hz, 1H); 7.92 ddd (J = 8, 2, 1Hz, 1H); 8.53 dd (J = 8, 2 Hz, 1H); 8.70 dd (J = 2, 1Hz, 1H).

### Beispiel 16

### 4-[[1-Phenyl-2-(3-pyridinyl)-1H-benzimidazol-6-yl]oxy]buttersäure

### a) 4-[[1-Phenyl-2-(3-pyridinyl)-1H-benzimidazol-6-yl]oxy]buttersäuremethylester

wurde durch Umsetzung von 6-Hydroxy-1-phenyl-2-(3-pyridinyl)-1*H-*benzimidazol mit 4-Brombutansäuremethylester gemäß der allgemeinen Arbeitsvorschrift 3 erhalten.
MS (EI): 387 (Molekülionpeak)

### b) 4-[[1-Phenyl-2-(3-pyridinyl)-1H-benzimidazol-6-yl]oxy]buttersäure

wurde durch Umsetzung von 4-[[1-Phenyl-2-(3-pyridinyl)-1*H-*benzimidazol-6-yl]oxy]buttersäuremethylester gemäß der allgemeinen Arbeitsvorschrift 4 erhalten.
MS (EI): 373 (Molekülionpeak)

### Vergleichs-Beispiel 17

### 6-[[1-Phenyl-2-(3-pyridinyl)-1H-benzimidazol-6-yl]oxy]hexansäure

### a) 6-[[1-Phenyl-2-(3-pyridinyl)-1H-benzimidazol-6-yl]oxy]hexansäuremethylester

wurde durch Umsetzung von 6-Hydroxy-1-phenyl-2-(3-pyridinyl)-1*H-*benzimidazol mit 6-Bromhexansäuremethylester gemäß der allgemeinen Arbeitsvorschrift 3 erhalten.
MS (EI): 415 (Molekülionpeak)

### b) 6-[[1-Phenyl-2-(3-pyridinyl)-1H-benzimidazol-6-yl]oxy]hexansäure

wurde durch Umsetzung von 6-[[1-Phenyl-2-(3-pyridinyl)-1*H-*benzimidazol-6-yl]oxy]hexansäuremethylester gemäß der allgemeinen Arbeitsvorschrift 4 erhalten.
MS (EI): 401 (Molekülionpeak)

### Vergleichs-Beispiel 18

### N-(3-Methoxypropyl)-6-[[1-(4-methylphenyl)-2-(3-pyridinyl)-1H-benzimidazol-6-yl]oxy]hexanamid

wurde durch Umsetzung von 6-[[1-(4-Methylphenyl)-2-(3-pyridinyl)-1*H-*benzimidazol-6-yl]oxy]hexansäuremethylester mit 3-Methoxypropylamin gemäß der allgemeinen Arbeitsvorschrift 6 dargestellt.
MS (EI): 486 (Molekülionpeak)

### Vergleichs-Beispiel 19

### 6-[[1-(4-Methylphenyl)-2-(3-pyridinyl)-1H-benzimidazol-6-yl]oxy]-1-morpholin-1-ylhexan-1-on

wurde durch Umsetzung von 6-[[1-(4-Methylphenyl)-2-(3-pyridinyl)-1*H-*benzimidazol-6-yl]oxy]hexansäuremethylester mit Morpholin gemäß der allgemeinen Arbeitsvorschrift 6 dargestellt.
MS (EI): 442 (Molekülionpeak)

### Vergleichs-Beispiel 20

### N-Methyl-6-[[1-(4-methylphenyl)-2-(3-pyridinyl)-1H-benzimidazol-6-yl]oxy] hexanamid

wurde durch Umsetzung von 6-[[1-(4-Methylphenyl)-2-(3-pyridinyl)-1*H-*benzimidazol-6-yl]oxy]hexansäuremethylester mit N-Methylaminhydrochlorid gemäß der allgemeinen Arbeitsvorschrift 6 dargestellt.
MS (EI): 428 (Molekülionpeak)

### Vergleichs-Beispiel 21

### N,N-Dimethyl-6-[[1-(4-methylphenyl)-2-(3-pyridinyl)-1H-benzimidazol-6-yl]oxy]hexanamid

wurde durch Umsetzung von 6-[[1-(4-Methylphenyl)-2-(3-pyridinyl)-1*H-*benzimidazol-6-yl]oxy]hexansäuremethylester mit Dimethylaminhydrochlorid gemäß der allgemeinen Arbeitsvorschrift 6 dargestellt.
MS (EI): 442 (Molekülionpeak)

### Vergleichs-Beispiel 22

### 6-[[1-(4-Methylphenyl)-2-(3-pyridinyl)-1H-benzimidazol-6-yl]oxy]hexanamid

wurde durch Umsetzung von 6-[[1-(4-Methylphenyl)-2-(3-pyridinyl)-1*H-*benzimidazol-6-yl]oxy]hexansäuremethylester mit Ammoniumchlorid gemäß der allgemeinen Arbeitsvorschrift 6 dargestellt.
MS (EI): 414 (Molekülionpeak)

### Vergleichs-Beispiel 23

### N-Cyclopropyl-6-[[1-(4-methylphenyl)-2-(-3-thienyl)-1H-benzimidazol-6-yl]oxy]hexanamid

wurde durch Umsetzung von 6-[[1-(4-Methylphenyl)-2-(3-thienyl)-1*H-*benzimidazol-6-yl]oxy]hexansäuremethylester mit Cyclopropylamin gemäß der allgemeinen Arbeitsvorschrift 6 dargestellt.
MS (EI): 459 (Molekülionpeak)

### Vergleichs-Beispiel 24

### N-Methyl-6-[[1-(4-methylphenyl)-2-(-3-thienyl)-1H-benzimidazol-6-yl]oxy] hexanamid

wurde durch Umsetzung von 6-[[1-(4-Methylphenyl)-2-(3-thienyl)-1*H-*benzimidazol-6-yl]oxy]hexansäuremethylester mit N-Methylaminhydrochlorid gemäß der allgemeinen Arbeitsvorschrift 6 dargestellt.
MS (EI): 433 (Molekülionpeak)

### Beispiel 25

### N-(2-Methoxyethyl)-5-[[1-(4-methylphenyl)-2-(3-pyridinyl)-1H-benzimidazol-6-yl]oxy]pentanamid

wurde durch Umsetzung von 5-[[1-(4-Methylphenyl)-2-(3-pyridinyl)-1*H-*benzimidazol-6-yl]oxy]pentansäuremethylester mit 2-Methoxyethylamin gemäß der allgemeinen Arbeitsvorschrift 6 dargestellt.
MS (EI): 458 (Molekülionpeak)

### Beispiel 26

### N,N-Dimethyl-5-[[1-(4-methylphenyl)-2-(3-pyridinyl)-1H-benzimidazol-6-yl]oxy]pentanamid

wurde durch Umsetzung von 5-[[1-(4-Methylphenyl)-2-(3-pyridinyl)-1*H-*benzimidazol-6-yl]oxy]pentansäuremethylester (Beispiel 56c) mit Dimethylamin gemäß der allgemeinen Arbeitsvorschrift 6 dargestellt.
MS (EI): 428 (Molekülionpeak)

### Beispiel 27

### 5-[[1-(4-Methylphenyl)-2-(3-pyridinyl)-1H-benzimidazol-6-yl]oxy]pentanamid

wurde durch Umsetzung von 5-[[1-(4-Methylphenyl)-2-(3-pyridinyl)-1*H-*benzimidazol-6-yl]oxy]pentansäuremethylester mit Ammoniumchlorid gemäß der allgemeinen Arbeitsvorschrift 6 dargestellt.
MS (EI): 400 (Molekülionpeak)

### Vergleichs-Beispiel 28

### 6-[[1-(4-Methylphenyl)-2-(2-thienyl)-1H-benzimidazol-6-yl]oxy]hexansäure

### a) 6-[[1-(4-Methylphenyl)-2-(2-thienyl)-1H-benzimidazol-6-yl]oxy]hexansäuremethylester

wurde durch Umsetzung von 6-[[4-Amino-3-((4-methylphenyl)amino)phenyl]-oxy]hexansäuremethylester mit 2-Thienylcarbaldehyd gemäß der allgemeinen Arbeitsvorschrift 5 erhalten.
MS (EI): 434 (Molekülionpeak)

### b) 6-[[1-(4-Methylphenyl)-2-(2-thienyl)-1H-benzimidazol-6-yl]oxy]hexansäure

wurde durch Umsetzung von 6-[[1-(4-Methylphenyl)-2-(2-thienyl)-1*H-*benzimidazol-6-yl]oxy]hexansäuremethylester gemäß der allgemeinen Arbeitsvorschrift 4 dargestellt.
MS (EI): 420 (Molekülionpeak)

### Beispiel 29

### Hemmung der Mikroglia-Aktivierung

Zur *in vitro* Darstellung von Aß-aktivierten Mikroglia werden primäre RattenMikroglia mit synthetischem Aß Peptid inkubiert:
Zur Simulierung von Aß-Ablagerungen wird synthetisches Aß Peptid auf 96-Loch Gewebekulturplatten eingetrocknet. Dazu wird eine Peptidstammlösung von 2mg/ml H₂O 1:50 in H₂O verdünnt. Zur Beschichtung der 96-Loch Platten werden 30µL dieser verdünnten Peptidlösung/Loch eingesetzt und über Nacht bei Raumtemperatur eingetrocknet.
Primäre Rattenmikroglia werden von gemischten Gliakulturen geerntet, die von P3 Rattenhirnen gewonnen wurden. Zu Herstellung von gemischten Gliakulturen werden die Hirne von 3 Tage alten Ratten entnommen und von Hirnhäuten befreit. Die Zellvereinzelung wird durch Trypsinisierung erreicht (0,25 % Trypsinlösung, 15 Min 37°C)). Nach Abtrennung von nicht-verdauten Gewebefragmenten mit Hilfe eines 40µm Nylonnetzes werden die isolierten Zellen abzentrifugiert (800 rpm/10 Min). Das Zellpellet wird in Kulturmedium resuspendiert und in 100ml Gewebekulturflaschen überführt. (1 Hirn/ Gewebekulturflasche). Die Kultivierung der Zellen erfolgt über einen Zeitraum von 5-7 Tagen in Dulbeccos modified Eagle Medium (DMEM, mit Glutamin), supplementiert mit Penicillin (50 U/ml), Streptomycin (40µg/ml) und 10 % (v/v) fötalem Kälber Serum (FCS) bei 37°C und 5% CO₂. Während dieser Inkubation wird ein adhäsiver Zellrasen gebildet, der hauptsächlich aus Astrozyten besteht. Mikroglia proliferieren als nicht- oder schwach-adhesive Zellen auf diesem und werden über Schüttelinkubation abgeerntet (420 Umdrehungen/Min, 1 Std).

Zur Aktivierung der Mikroglia durch Aß-Peptid werden 2,5 mal 10⁴ Mikroglia/Loch auf Aß-beschichtete Gewebekulturplatten ausgesät und über einen Zeitraum von 7 Tagen in DMEM (mit Glutamin), supplementiert mit Penicillin (50 U/ml), Streptomycin (40µg/ml) und 10 % (v/v) fötalem Kälber Serum (FCS) bei 37°C und 5% CO₂ inkubiert. Am Tag 5 erfolgt die Zugabe einer erfindungsgemäßen Verbindung in verschiedenen Konzentrationen (0,1, 0,3,1,3, und 10µM).
Zur Quantifizierung der Mikroglia-Reaktivität wird am Kultivierungstag 7 die metabolische Aktivität über die Reduktion von MTS (3-(4,5-dimethylthiazol-2-yl)-5-(3-carboxymethoxyphenyl)-2-(sulfophenyl)-2H-tetrazolium), Owen's Reagenz, Baltrop, J.A. et al. Bioorg. & Med. Chem. Lett 1, 6111 (1991)) gemessen. Die Prozent Inhibition bezieht sich auf eine nur mit DMSO behandelte Kontrolle. Die erfindungsgemäßen Verbindungen inhibieren die Mikroglia-Aktivierung

### Beispiel 30

### Zerebraler Hirninfarkt in der Ratte (MCAO-Modell)

Die erfindungsgemäßen Verbindungen wurden in einem Tiermodell für zerebrale Ischämie (Schlaganfall), dem MCAO (permanent middle cerebral artery occlusion) Modell, auf *in vivo* Aktivität hin getestet. Durch einseitigen Verschluß der mittleren Hirnarterie (MCA) wird ein Hirninfarkt ausgelöst, der auf der Unterversorgung des entsprechenden Hirnbereiches mit Sauerstoff und Nährstoffen beruht. Folge dieser Unterversorgung ist ein ausgeprägter Zelluntergang sowie, nachfolgend, eine starke Mikroglia-Aktivierung. Diese Mikroglia-Aktivierung erreicht allerdings erst nach mehreren Tagen ihr Maximum und kann über mehrere Wochen anhalten. Zur Testung der Substanzen wurden die erfindungsmäßigen Verbindungen 1-6 Tage nach Okklusion intraperitonal appliziert. Die Tiere wurden am Tag 7 perfundiert und getötet. Das Ausmaß der Mikroglia-Aktivierung wurde durch eine modifizierte immunhistochemische Methode gemessen. Dazu wurden Vibratom-Schnitte von fixierten Gehirnen mit Antikörper inkubiert, die den CR3 Komplement-Rezeptor bzw den MHCII Komplex auf aktivierten Mikroglia erkennen. Die Quantifizierung der primären Antikörperbindung erfolgte durch eine Enzym-gekoppeltes Detektionssystem. Die Behandlung mit den erfindungsgemäßen Verbindungen führte zu einer Reduktion der Mikroglia-Aktivierung in der vom Hirninfarkt betroffenen Hirnhemisphere.

### Beispiel 31

### Inhibition der TNFα und IL 12 Produktion in THP-1 Zellen

Die Inhibition der Zytokin-Produktion wird beispielsweise durch Messung von TNFα und Interleukin 12 in Lipopolysaccharid (LPS) stimulierten THP-1 Zellen dargestellt.
Dazu werden 2,5 x10⁶ THP-1 Zellen (American Type Culture Company, Rockville, Md)/ mL RPMI 1640 Medium (Life technologies)/10%FCS (Life Technologies, Kat.Nr. 10270-106) auf 96 Loch - Flachboden Zellkulturplatten (TPP, Produkt Nr. 9296) ausgesät (100 µL/Loch) Die erfindungsmäßigen Verbindungen werden in unterschiedlichen Konzentrationen hinzugegeben und für 30 Minuten vorinkubiert. Die Vorverdünnung der Testsubstanzen wurde in Inkubationsmedium durchgeführt. Die Zugabe der Testsubstanzen erfolgt als 2 fach konzentrierte Substanzlösung (100 µl(Loch). Die Stimulation der Zellen erfolgte über Nacht bei 37°C mit 0,1µg/ml LPS (Sigma L2630, von E. Coli Serotyp 0111. B4). Danach wurde das Medium geerntet und die TNFα bzw. die Interleukin 12-Menge quantitativ bestimmt. Zur Messung der TNFα wurde ein kommerziell erhältlicher TNFα Kit der Firma *CIS bio international* eingesetzt (Produkt Nr. 62TNFPEB). Die Interleukin 12 Menge wurde mit Hilfe der ORIGEN Technologie (IGEN International, Inc, Gaithersburg, Maryland) durchgeführt Der errechnete IC₅₀ Wert entspricht der Konzentration an Testsubstanz, die benötigt wird, um eine 50%ige Inhibition der maximalen TNFα bzw Interleukin 12 Produktion zu erzielen.
Die erfindungsgemäßen Verbindungen inhibieren die TNFα- und Interleukin 12-Produktion in Lipopolysaccharid (LPS) stimulierten THP-1 Zellen.

### Beispiel 32

### Inhibition der INFγ Produktion von peripheren mononukleären Blutzellen

Zur Darstellung des Einflußes der Substanzen auf die T-Zell-Aktivierung wird beispielsweise die Messung der INFγ Sekretion eingesetzt.
Zur Isolation von periphere mononukleäre Zellen wurde humanes Vollblut verwendet (Blutabnahme via Na-Zitrat S-Monovetten "Coagulation 9 NC/10ml"/ Sarstedt). Die Anreicherung der Blutzellen wurde mit Hilfe der Dichtegradientenzentrifugation durchgeführt: Dazu werden 15 ml Histopaque 1077 Sigma, Kat. Nr. H8880) in LEUCOSEP Röhrchen (Greiner, Kat. Nr. 227290) vorgelegt und 30 Sekunden bei 1000g zentrifugiert. Anschließend werden 15 ml Vollblut hinzugegeben und 10 Min bei 1000g zentrifugiert. Abschließend wird die obere Plasmaschicht abpipettiert und darunter liegende Zellschicht (periphere mononukleäre Blutzellen) in 15 ml Probenröhrchen (Falcon ) übertragen und anschließend mehrmals mit 10 ml HBSS HANKS balanced Solution (ohne Mg²⁺ und Ca²⁺), Kat. Nr. 14175-53) gewaschen. Zuletzt wird das Zellpellet in Kulturmedium RPMI 1640 +25 mM Hepes (Life Technologie Kat. Nr. 52400-04110% FCS (Life Technologies, Kat.Nr. 10270-106), 0,4% Penicillin-Streptomycin Lösung (Life Technologies , Kat. Nr. 15140-106) resuspendiert (1 x 10⁶ Zellen/ml). Jeweils 100 µl Zellsuspensionlösung wurden auf 96 Loch-Flachboden-Zellkulturplatten (TPP, Produkt Nr. 9296) verteilt und mit 2,5 µg/ml anti-CD3 Antikörper stimuliert. Die erfindungsmäßigen Substanzen wurden in unterschiedlichen Konzentrationen hinzugegeben und für 30 Minuten vorinkubiert. Die Stimulation der Zellen erfolgte über einen Zeitraum von 24 h. Danach wurde das Mediium geerntet und die INFγ quantitativ bestimmmt. Die INFγ Menge wurde mit Hilfe der ORIGEN Technologie (IGEN International, Inc, Gaithersburg, Maryland) durchgeführt Der errechnete IC₅₀ Wert entspricht der Konzentration an Testsubstanz, die benötigt wird, um eine 50%ige Inhibition der maximalen INFγ Produktion zu erzielen.

Die erfindungsgemäßen Verbindungen inhibieren die INFγ Produktion von peripheren mononukleären Blutzellen.

### Beispiel 33

### Inhibition der TNFα und IL-12 HD-Produktion von peripheren mononukleären Blutzellen

Die Inhibition der TNFα und IL-12 HD p70-Produktion wird beispielsweise durch Messung von TNFα und IL-12 HD p70 in mit Lipopolysaccharid (LPS) und Interferon gamma (IFNγ) stimulierten peripheren mononukleären Blutzellen dargestellt.

Zur Isolation von peripheren mononukleären Blutzellen wurde humanes Vollblut verwendet (Blutabnahme via Na-Zitrat S-Monovetten "Coagulation 9 NC/10ml"/ Sarstedt). Die Anreicherung der Lymphozyten und Monozyten wurde mit Hilfe der Dichtegradientenzentrifugation durchgeführt: Dazu werden 15 ml Histopaque-1077 (Sigma, Kat. Nr. H8880) in 50 ml LEUCOSEP Röhrchen (Greiner, Kat. Nr. 227290) vorgelegt und durch Zentrifugation für 30 Sekunden bei 250g durch die in den Röhrchen befindliche Fritte nach unten gedrückt. Anschließend werden 20 ml Vollblut hinzugegeben und 15 Min. bei 800g und Raumtemperatur zentrifugiert. Nach der Zentrifugation wird der Überstand (Plasma und Thrombozyten) abpipettiert und verworfen und die darunter liegende Zellschicht (Lymphozyten und Monozyten) in 50 ml Zentrifugenröhrchen (Falcon) übertragen und anschließend 3x in Kulturmedium VLE RPMI 1640 (Seromed, No. FG1415) gewaschen (Zentrifugation jeweils 10 Min. bei 250g, Raumtemperatur). Zuletzt wird das Zellpellet in Kulturmedium VLE RPMI 1640 (Seromed, No. FG1415), 10% FCS (Life Technologies, Kat. Nr. 16000-044, low endotoxin, Hitze-inaktiviert 1h, 56°C), 50 µg/ml Penicillin-Streptomycin Lösung (Life Technologies, Kat. Nr. 15140-106) resuspendiert und nach Zellzählung mittels Trypanblaufärbung auf 3 x 10⁶ Zellen/ml eingestellt. Jeweils 100 µl Zellsuspensionslösung wurden auf 96 Loch-Flachboden-Zellkulturplatten (Costar, Produkt Nr. 3599) verteilt. Dazugegeben wurden jeweils 100 µl 3fach-konzentrierte Stimulationslösung (3 µg/ml LPS von *E. coli* Serotyp 0127:B8; Sigma, Kat-No. L-4516 und 30 ng/ml IFNγ 1b, Imukin, Boehringer Ingelheim). Die erfindungsmäßigen Substanzen wurden in unterschiedlichen Konzentrationen als 3fach-konzentrierte Substanzlösung (100 µl/well) hinzugegeben. Die Stimulation der Zellen erfolgte bei 37°C und 5% CO₂ über einen Zeitraum von 24 h. Danach wurde der Zellkulturüberstand geerntet und die Konzentrationen von TNFα und IL-12 HD p70 mittels kommerziell erhältlicher ELISA-Kits der Firmen *BioSource International* (TNF-α EASIA, Kat. Nr. KAC1752) und *R&D Systems* (Quantikine™ HS IL-12, Kat. Nr. HS 120) bestimmt.
Der errechnete IC₅₀ Wert entspricht der Konzentration an Testsubstanz, die benötigt wird, um eine 50%ige Inhibition der maximalen TNFα- bzw. Interleukin 12 HD p70-Produktion zu erzielen.
Die erfindungsgemäßen Verbindungen inhibieren die TNFα und IL-12 HD p70-Produktion von peripheren mononukleären Blutzellen.

### Beispiel 34

### Induktion der IL-10-Produktion von peripheren mononukleären Blutzellen

Die Induktion der IL-10-Produktion wird beispielsweise durch Messung von IL-10 in in Phytohämagglutinin (PHA) oder Lipopolysaccharid (LPS) stimulierten peripheren mononukleären Blutzellen dargestellt.
Zur Isolation von peripheren mononukleären Blutzellen wurde humanes Vollblut verwendet (Blutabnahme via Na-Zitrat S-Monovetten "Coagulation 9 NC/10ml"/ Sarstedt). Die Anreicherung der Lymphozyten und Monozyten wurde mit Hilfe der Dichtegradientenzentrifugation durchgeführt: Dazu werden 15 ml Histopaque-1077 (Sigma, Kat. Nr. H8880) in 50 ml LEUCOSEP Röhrchen (Greiner, Kat. Nr. 227290) vorgelegt und durch Zentrifugation für 30 Sekunden bei 250g durch die in den Röhrchen befindliche Fritte nach unten gedrückt. Anschließend werden 20 ml Vollblut hinzugegeben und 15 Min. bei 800g und Raumtemperatur zentrifugiert. Nach der Zentrifugation wird der Überstand (Plasma und Thrombozyten) abpipettiert und verworfen und die darunter liegende Zellschicht (Lymphozyten und Monozyten) in 50 ml Zentrifugenröhrchen (Falcon) übertragen und anschließend 3x in Kulturmedium VLE RPMI 1640 (Seromed, No. FG1415) gewaschen (Zentrifugation jeweils 10 Min. bei 250g, Raumtemperatur). Zuletzt wird das Zellpellet in Kulturmedium VLE RPMI 1640 (Seromed, No. FG1415), 10% FCS (Life Technologies, Kat. Nr. 16000-044, low endotoxin, Hitze-inaktiviert 1h, 56°C), 50 µg/ml Penicillin-Streptomycin Lösung (Life Technologies, Kat. Nr. 15140-106) resuspendiert und nach Zellzählung mittels Trypanblaufärbung auf 3 x 10⁶ Zellen/ml eingestellt. Jeweils 100 µl Zellsuspensionslösung wurden auf 96 Loch-Flachboden-Zellkulturplatten (Costar, Produkt Nr. 3599) verteilt. Dazugegeben wurden jeweils 100 µl 3fach-konzentrierte Stimulationslösung (3 µg/ml LPS von *E. coli* Serotyp 0127:B8; Sigma, Kat.Nr. L-4516 bzw. 300 µg/ml PHA-L, Biochrom KG, Kat.Nr. M5030). Die erfindungsmäßigen Substanzen wurden in unterschiedlichen Konzentrationen als 3fach-konzentrierte Substanzlösung (100 µl/well) hinzugegeben. Die Stimulation der Zellen erfolgte bei 37°C und 5% CO₂ über einen Zeitraum von 24 h. Danach wurde der Zellkulturüberstand geerntet und IL-10 quantitativ bestimmmt. Die IL-10-Konzentration wurde mittels eines kommerziell erhältlichen ELISA-Kits der Firma *BioSource International* bestimmt (Human IL-10, Kat.Nr. KHC0101C). Der errechnete EC₅₀ Wert entspricht der Konzentration an Testsubstanz, die benötigt wird, um die IL-10-Sekretion um 50% der maximalen Steigerung zu erhöhen.
Die erfindungsgemäßen Verbindungen steigern die IL-10-Produktion von peripheren mononukleären Blutzellen.

## Patentansprüche

1. Benzimidazolderivate der allgemeinen Formel I 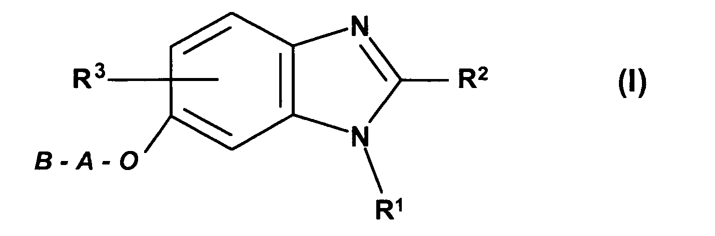 worin
R¹ eine Phenylgruppe, die gegebenenfalls mit bis zu drei folgenden Substituenten unabhängig voneinander substituiert ist:
F, Cl, Br, J
OH, OR⁴, OCOR⁴
SR⁴, SOR⁴, SO₂R⁴,
R⁴,
NH₂, NHR⁴, NR⁴R⁴'
oder zwei benachbarte Substituenten an R¹ bilden zusammen eine -O-CH₂-O-, -O- CH₂-CH₂-O- oder -CH₂-CH₂-CH₂- Gruppe,
R² eine monocyclische oder bicyclische 5 - 10-gliedrige Heteroarylgruppe mit 1 - 2 Heteroatomen ausgewählt aus N, S und O, die gegebenenfalls mit bis zu zwei der folgenden Substituenten unabhängig voneinander substituiert ist:
F, Cl, Br, J
OH, OR⁴, OCOR⁴,
COR⁴,
SR⁴, SOR⁴, SO2R⁴,
R⁴
oder zwei benachbarte Substituenten an R² bilden zusammen eine -O-CH₂-O-, -O-CH₂-CH₂-O- oder -CH₂-CH₂-CH₂- Gruppe,
R³ H, OH oder O-C₁₋₆-Alkyl,
R⁴ und R^{4,} unabhängig voneinander C₁₋₄-Perfluoralkyl oder C₁₋₆-Alkyl,
A eine geradkettige Alkylengruppe, ausgewählt aus Ethylen, Propylen und Butylen, die gegebenenfalls mit =O, OH,
O-C₁₋₃-Alkyl, NH₂, NH-C₁₋₃-Alkyl, NH-C₁₋₃-Alkanoyl, N(C₁₋₃-Alkyl)₂, und N(C₁₋₃- Alkyl)(C₁₋₃-Alkanoyl) substituiert ist,
B COOH, CONH₂, CONHNH₂, CONHR⁵, CONR⁵R^{5'}, jeweils gebunden an ein Kohlenstoffatom der Gruppe A,
R⁵ und R^{5'} unabhängig voneinander jeweils ein Rest, ausgewählt aus der Gruppe umfassend C₁₋₆-Alkyl, C₂₋₆-Alkenyl, C₂₋₆-Alkinyl,
wobei ein C-Atom gegen O, S, SO, SO₂, NH, N-C₁₋₃-Alkyl oder N-C₁₋₃-Akaloyl ausgetauscht sein kann, ferner (C₀₋₃-Alkandiyl-C₃₋₇Cycloalkyl), wobei in einem fünfgliedrigen Cycloalkylring ein Ringglied Ring-N oder Ring-O sein kann und in einem sechs- oder siebengliedrigen Cycloalkylring ein oder zwei Ringglieder jeweils Ring-N- und/oder Ring-O-Atome sein können, wobei die Ring-N-Atome gegebenenfalls mit C₁₋₃-Alkyl oder C₁₋₃Alkanoyl substituiert sein können, sowie ferner (C₀₋₃-Alkandiyl-Phenyl) und
(C₀₋₃-Alkandiyl-Heteroaryl), wobei die Heteroarylgruppe fünf- oder sechsgliedrig ist und ein oder zwei Heteroatome enthält, ausgewählt aus der Gruppe umfassend N, S und O,
wobei alle zuvor genannten Alkyl- und Cycloalkylreste gebenenfalls mit bis zu zwei Resten, ausgewählt aus der Gruppe umfassend CF₃, C₂F₅, OH, O-C₁₋₃-Alkyl, NH₂, NH-C₁₋₃-Alkyl, NH-C₁₋₃-Alkanoyl, N(C₁₋₃-Alkyl)₂, N(C₁₋₃-Alkyl)(C₁₋₃-Alkanoyl), COOH, CONH₂ und COO-C₁₋₃-Alkyl, und alle zuvor genannten Phenyl- und Heteroarylgruppen, gegebenenfalls mit bis zu zwei Resten, ausgewählt aus der Gruppe umfassend F, Cl, Br, CH₃, C₂H₅, OH, OCH₃, OC₂H₅, NO₂, N(CH₃)₂, CF₃, C₂F₅ und SO₂NH₂ substituiert sein können
oder R⁵ und R^{5'} gemeinsam mit dem N-Atom einen fünf- bis siebengliedrigen heterocyclischen Ring bilden, der ein weiteres N- oder O- oder S-Atom enthalten kann und der substituiert sein kann mit C₁₋₄-Alkyl, (C₀₋₂-Alkandiyl-C₁₋₄-Alkoxy), C₁₋₄- Alkoxycarbonyl, Aminocarbonyl oder Phenyl
bedeuten,
sowie deren optische oder geometrische isomere oder tautomere Formen oder pharmazeutisch anwendbare Salze.

2. Benzimidazole nach Anspruch 1 **dadurch gekennzeichnet, dass**
R¹ eine Phenylgruppe ist, die gegebenenfalls mit bis zu zwei der folgenden Substituenten unabhängig voneinander substituiert ist:
F, Cl,
OH, OR⁴, OCOR⁴
SR⁴,
R⁴ oder
zwei benahbarte Substituenten an R¹ bilden eine -O-CH₂-O- oder -CH₂-CH₂-CH₂- Gruppe.

3. Benzimidazole nach Anspruch 1 oder 2 **dadurch gekennzeichnet, dass**
R² eine monocyclische 5-6 gliedrige Heteroarylgruppe mit 1-2 Heteroatomen ausgewählt aus der Gruppe umfassend N, S und O ist, die gegebenenfalls mit bis zu zwei der folgenden Substituenten unabhängig substituiert ist:
F, Cl,
OR⁴, OCOR⁴
SR⁴, SOR⁴,SO₂R⁴,
R⁴ oder
zwei benachbarte Substituenten an R² bilden eine -O-CH₂-O- oder CH₂-CH₂-CH₂- Gruppe.

4. Benzimidazole nach einem der Ansprüche 1-3 **dadurch gekennzeichnet, dass** R³ H ist.

5. Benzimidazole nach einem der Ansprüche 1-4 **dadurch gekennzeichnet, dass** R⁴ und R⁴' unabhängig voneinander C₁₋₂ Perfluoralkyl, C₁₋₄ Alkyl sind.

6. Benzimidazole nach einem der Ansprüche 1-5 **dadurch gekennzeichnet, dass**
R⁵ und R^{5,} unabhängig voneinander C₁₋₆ Alkyl sind, wobei ein Kohlenstoffatom gegen O, S, SO, SO² ausgetauscht sein kann,
C₃₋₅ Cycloalkyl-C₀₋₃ Alkylen, wobei in einem 5-gliedrigen Cycloalkylring ein Ringglied ein N oder ein O sein kann, wobei der Ringstickstoff gegebenenfalls mit C₁₋₃ Alkyl oder C₁₋₃ Alkanoyl substituiert ist,
C₀₋₂ Alkylen-(5-6 gliedriges Heteroaryl mit 1-2 Heteroatomen aus N, S und O) bedeuten,
wobei alle zuvor genannten Alkyl- und Cycloalkylreste mit CF₃, OH, NH2, NH C₁₋₃ Alkyl, NH C₁₋₃ Alkanoyl, N(C₁₋₃Alkyl)₂, N(C₁₋₃Alkyl)(C₁₋₃ Alkanoyl), COOH,CONH₂ und alle zuvor genannten Heteroarylgruppen mit ein oder zwei Substituenten aus der Gruppe bestehend aus F, Cl, CH₃, C₂H₅, OCH₃, OC₂H₅, CF₃, C₂F₅ substituiert sein können,
oder R⁵ und R^{5'} gemeinsam mit dem Stickstoffatom einen 5-7 gliedrigen Heterocyclus bilden, der ein weiteres Sauerstoff-, Stickstoff- oder Schwefelatom enthalten kann und substituiert sein kann mit C₁₋₄-Alkyl, C₁₋₄-Alkoxy-C₀₋₂-alkyl.

7. Benzimidazole nach einem der Ansprüche 1-5, **dadurch gekennzeichnet, dass**
B COOH oder CONH₂
jeweils gebunden an ein Kohlenstoffatom der Gruppe A ist.

8. Eine Verbindung nach Anspruch 1, ausgewählt aus der Gruppe bestehend aus
5-[[1-(4-Methylphenyl)-2-(3-pyridinyl)-1H-benzimidazol-6-yl]oxy]pentansäure
4-[[1-(4-Methylphenyl)-2-(3-pyridinyl)-1H-benzimidazol-6-yl]oxy]buttersäure
5-[[1-(4-Methylphenyl)-2-(3-thienyl)-1H-benzimidazol-6-yl]oxy]pentansäure
4-[[1-(4-Methylphenyl)-2-(3-thienyl)-1H-benzimidazol-6-yl]oxy]buttersäure
5-[[1-Phenyl-2-(3-thienyl)-1H-benzimidazol-6-yl]oxy]pentansäure
4-[[1-Phenyl-2-(3-thienyl)-1H-benzimidazol-6-yl]oxy]buttersäure
5-[[1-(4-Fluorphenyl)-2-(3-thienyl)-1H-benzimidazol-6-yl]oxy]pentansäure
5-[[1-(4-Fluorphenyl)-2-(3-pyridinyl)-1H-benzimidazol-6-yl]oxy]pentansäure
5-[[1-Phenyl-2-(3-pyridinyl)-1H-benzimidazol-6-yl]oxy]pentansäure
4-[[1-Phenyl-2-(3-pyridinyl)-1H-benzimidazol-6-yl]oxy]buttersäure
N-(2-Methoxyethyl)-5-[[1-(4-methylphenyl)-2-(3-pyridinyl)-1H-benzimidazol-6-yl]oxy]pentanamid
N,N-Dimethyl-5-[[1-(4-methylphenyl)-2-(3-pyridinyl)-1H-benzimidazol-6-yl]oxy] pentanamid
5-[[1-(4-Methylphenyl)-2-(3-pyridinyl)-1H-benzimidazol-6-yl]oxy]pentanamid.

9. Verwendung einer Verbindung nach einem der Ansprüche 1-8 zur Herstellung eines Arzneimittels zur Behandlung oder Prevention von Erkrankungen, die mit einer Mikroglia-Aktivierung assoziiert sind.

10. Verwendung nach Anspruch 9 zur Behandlung oder Prevention von inflammatorischen, allergischen, infektiösen oder autoimmunen Erkrankungen.

11. Pharmazeutische Mittel, die **dadurch gekennzeichnet sind, dass** sie eine oder mehrere Verbindungen nach einem der Ansprüche 1-8 und einen oder mehrere Träger- und/oder Hilfsstoffe enthalten.

## Claims

1. Benzimidazole derivatives of the general formula I 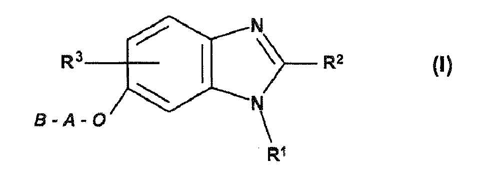 in which
R¹ is a phenyl group which is optionally substituted with up to three of the following substituents independently of one another,
F, Cl, Br, J
OH, OR⁴, OCOR⁴ SR⁴, SOR⁴, SO₂R⁴,
SR⁴, SOR⁴, SO₂R⁴,
R⁴,
NH₂, NHR⁴, NR⁴R⁴',
or two adjacent substituents on R¹ together form a -O-CH₂-O-, -O-CH₂-CH₂-O- or -CH₂-CH₂-CH₂ group,
R² is a monocyclic or bicyclic 5-10-membered heteroaryl group with 1-2 heteroatoms selected from N, S and 0, which is optionally substituted with up to two of the following substituents independently of one another:
F, Cl, Br, J OR⁴, OCOR⁴,
OH, OR⁴, OCOR⁴,
COR⁴,
SR⁴, SOR⁴, SO₂R⁴, SR⁴, SOR⁴, SO₂R⁴,
R⁴
or two adjacent substituents on R² together form a -O-CH₂-O-, -O-CH₂-CH₂-O- or -CH₂-CH₂-CH₂ group,
R³ is H, OH or O-C₁₋₆-alkyl,
R⁴ and R⁴', independently of one another, are C₁₋₄- perfluoroalkyl or C₁₋₆-alkyl,
A is a straight-chain alkylene group selected from ethylene, propylene and butylene which is optionally substituted with =O, OH, O-C₁₋₃-alkyl, NH₂, NH-C₁₋₃-alkyl, NH-C₁₋₃-alkanoyl, N(C₁₋₃-alkyl)₂, and N(C₁₋₃-alkyl)(C₁₋₃alkanoyl),
B is COOH, CONH₂, CONHNH₂, CONHR⁵, CONR⁵R⁵', in each case bonded to a carbon atom of group A,
R⁵ and R⁵', independently of one another, are in each case a radical selected from the group comprising C₁₋₆-alkyl, C₂₋₆-alkenyl, C₂₋₆-alkinyl, where one carbon atom can be exchanged for 0, S, SO, SO₂, NH, N-C₁₋₃-alkyl or N-C₁₋₃-alkanoyl, also (C₀₋₃- alkanediyl-C₃₋₇-cycloalkyl), where, in a five- membered cycloalkyl ring, one ring member can be ring N or ring 0, and, in a six- or seven-membered cycloalkyl ring, one or two ring members can in each case be ring N and/or ring 0 atoms, where the ring N atoms can be optionally substituted with C₁₋₃-alkyl or C₁₋₃-alkanoyl, and in addition (C₀₋₃- alkanediyl-phenyl) and
(C₀₋₃-alkanediyl-heteroaryl), where the heteroaryl group is five- or six-membered and contains one or two heteroatoms selected from the group comprising N, S and 0,
where all of the aforementioned alkyl and cycloalkyl radicals can be optionally substituted with up to two radicals selected from the group comprising CF₃, C₂F₅, OH, O-C₁₋₃-alkyl, NH₂, NH-C₁₋₃- alkyl, NH-C₁₋₃-alkanoyl, N(C₁₋₃-alkyl)₂, N(C₁₋₃- alkyl)(C₁₋₃-alkanoyl), COOH, CONH₂ and COO-C₁₋₃- alkyl, and all of the aforementioned phenyl and heteroaryl groups, optionally with up to two radicals selected from the group comprising F, Cl, Br, CH₃, C₂H₅, OH, OCH₃ OC₂H₅, NO₂, N(CH₃)₂, CF₃, C₂F₅ and SO₂NH₂
or R⁵ and R⁵', together with the N atom, form a five- to seven-membered heterocyclic ring which can contain a further N or 0 or S atom and which can be substituted with C₁₋₄-alkyl, (C₀₋₂- alkanediyl-C₁₋₄-alkoxy), C₁₋₄-alkoxycarbonyl, aminocarbonyl or phenyl,
and their optical or geometric isomeric or tautomeric forms or pharmaceutically applicable salts.

2. Benzimidazoles according to Claim 1, **characterized in that**
R¹ is a phenyl group which is optionally substituted with up to two of the following substituents independently of one another:
F, Cl,
OH, OR⁴, OCOR⁴,
SR⁴,
R⁴, or
two adjacent substituents on R¹ form a -O-CH₂-O- or -CH₂-CH₂-CH₂ group.

3. Benzimidazoles according to Claim 1 or 2, **characterized in that**
R² is a monocyclic 5-6-membered heteroaryl group with 1-2 heteroatoms selected from the group comprising N, S and 0, which is optionally substituted with up to two of the following substituents independently,
F, Cl,
OR⁴ OCOR⁴
SR⁴, SOR⁴, SO₂R⁴,
R⁴ or
two adjacent substituents on R² form a -O-CH₂-O- or -CH₂-CH₂-CH₂ group.

4. Benzimidazoles according to one of Claims 1-3, **characterized in that** R³ is H.

5. Benzimidazoles according to one of Claims 1-4, **characterized in that**
R⁴ and R⁴', independently of one another, are C₁₋₂ perfluoroalkyl, C₁₋₄ alkyl.

6. Benzimidazoles according to one of Claims 1-5, **characterized in that**
R⁵ and R⁵', independently of one another, are C₁₋₆ alkyl, where one carbon atom can be exchanged for 0, S, SO, SO²,
C₃₋₅ cycloalkyl-C₀₋₃ alkylene where, in a 5-membered cycloalkyl ring, one ring member can be an N or an 0, where the ring nitrogen is optionally substituted with C₁₋₃ alkyl or C₁₋₃ alkanoyl,
C₀₋₂ alkylene-(5-6-membered heteroaryl with 1-2 heteroatoms from N, S and 0),
where all of the aforementioned alkyl and cycloalkyl radicals can be substituted with CF₃, OH, NH₂, NHC₁₋₃ alkyl, NHC₁₋₃ alkanoyl, N(C₁₋₃ alkyl)₂, N(C₁₋₃ alkyl)(C₁₋₃ alkanoyl), COOH, CONH₂ and all of the aforementioned heteroaryl groups can be substituted with one or two substituents from the group consisting of F, Cl, CH₃, C₂H₅, OCH₃, OC₂H₅, CF₃, C₂F₅,
or R⁵ and R⁵', together with the nitrogen atom, form a 5-7-membered heterocycle which can contain a further oxygen, nitrogen or sulphur atom and can be substituted with C₁₋₄-alkyl, C₁₋₄-alkoxy-C₀₋₂- alkyl.

7. Benzimidazoles according to one of Claims 1-5, **characterized in that**
B is COOH or CONH₂
in each case bonded to a carbon atom of group A.

8. A compound according to Claim 1, selected from the group consisting of
5-[[1-(4-methylphenyl)-2-(3-pyridinyl)-1H-benzimidazol-6-yl]oxy]pentanoic acid
4-[[1-(4-methylphenyl)-2-(3-pyridinyl)-1H-benzimidazol-6-yl]oxy]butyric acid
5-[[1-(4-methylphenyl)-2-(3-thienyl)-1H-benzimidazol-6-yl]oxy]pentanoic acid
4-[[1-(4-methylphenyl)-2-(3-thienyl)-1H-benzimidazol-6-yl]oxy]butyric acid
5-[[1-phenyl-2-(3-thienyl)-1H-benzimidazol-6-yl]oxy]pentanoic acid
4-[[1-phenyl-2-(3-thienyl)-1H-benzimidazol-6-yl]oxy]butyric acid
5-[[1-(4-fluorophenyl)-2-(3-thienyl)-1H-benzimidazol-6-yl]oxy]pentanoic acid
5-[[1-(4-fluorophenyl)-2-(3-pyridinyl)-1H-benzimidazol-6-yl]oxy]pentanoic acid
5-[[1-phenyl-2-(3-pyridinyl)-1H-benzimidazol-6-yl]oxy]pentanoic acid
4-[[1-phenyl-2-(3-pyridinyl)-1H-benzimidazol-6-yl]oxy]butyric acid
N-(2-methoxyethyl)-5-[[1-(4-methylphenyl)-2-(3-pyridinyl)-1H-benzimidazol-6-yl]oxy]pentanamide
N,N-dimethyl-5-[[1-(4-methylphenyl)-2-(3-pyridinyl)-1H-benzimidazol-6-yl]oxy]pentanamide
5-[[1-(4-methylphenyl)-2-(3-pyridinyl)-1H-benzimidazol-6-yl]oxy]pentanamide.

9. Use of a compound according to one of Claims 1-8 for producing a drug for treating or preventing diseases associated with a microglia activation.

10. Use according to Claim 9 for treating or preventing inflammatory, allergic, infectious or autoimmune diseases.

11. Pharmaceutical compositions which are **characterized in that** they comprise one or more compounds according to one of Claims 1-8 and one or more carriers and/or excipients.

## Revendications

1. Dérivés de benzimidazole de formule générale I 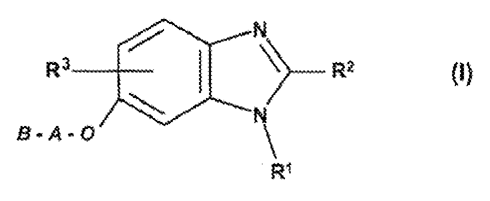 dans laquelle
R¹ est un groupe phényle, qui est éventuellement substitué par jusqu'à trois des substituants suivants, indépendamment les uns des autres :
F, Cl, Br, I
OH, OR⁴, OCOR⁴
SR⁴, SOR⁴, SO₂R⁴
R⁴,
NH₂, NHR⁴, NR⁴R^{4'}
ou deux substituants voisins sur R¹ forment ensemble un groupe -O-CH₂-O-, -O-CH₂-CH₂-O-, ou -CH₂-CH₂-CH₂-,
R² est un groupe hétéroaryle monocyclique ou bicyclique à 5-10 chaînons, ayant 1-2 hétéroatomes choisis parmi N, S et 0, qui éventuellement sont substitués par jusqu'à deux des substituants suivants, indépendamment les uns des autres :
F, Cl, Br, I
OH, OR⁴, OCOR⁴,
COR⁴,
SR⁴, SOR⁴, SO₂R⁴,
R⁴
ou deux substituants voisins sur R² forment ensemble un groupe -O-CH₂-O-, -O-CH₂-CH₂-O- ou -CH₂- CH₂-CH₂-,
R³ est H, OH ou un groupe O- (alkyle en C₁₋₆),
R⁴ et R^{4'} représentent chacun indépendamment de l'autre un radical perfluoralkyle en C₁₋₄ ou alkyle en C₁₋₆,
A est un groupe alkylène à chaîne droite, choisi parmi l'éthylène, le propylène et le butylène, qui est éventuellement substitué par un ou des substituants =O, OH, O-(alkyle en C₁₋₃), NH₂, NH- (alkyle en C₁₋₃), NH-(alcanoyle en C₁₋₃), N(alkyle en C₁₋₃)₂ et N(alkyle en C₁₋₃)(alcanoyle en C₁₋₃),
B est COOH, CONH₂, CONHNH₂, CONHR⁵, CONR⁵R^{5'}, chacun d'eux étant lié à un atome de carbone du groupe A,
R⁵ et R^{5'} représentent chacun indépendamment de l'autre un radical choisi dans le groupe comprenant les groupes alkyle en C₁₋₆, alcényle en C₂₋₆, alcynyle en C₂₋₆, où un atome de carbone peut être remplacé par 0, S, SO, SO₂, NH, un radical N-(alkyle en C₁₋₃) ou N-(alcanoyle en C₁₋₃), et en outre (alcanediyle en C₀₋₃)-(cycloalkyle en C₃₋₇), où, dans un noyau cycloalkyle à cinq chaînons, un chaînon cyclique peut être un atome d'azote cyclique ou d'oxygène cyclique et, dans un noyau cycloalkyle à six ou sept chaînons, un ou deux chaînons cycliques peuvent être chacun des atomes d'azote cyclique et/ou des atomes d'oxygène cyclique, les atomes d'azote cyclique pouvant éventuellement être substitués par un ou des substituants alkyle en C₁- ₃ ou alcanoyle en C₁₋₃, et en outre ((alcanediyle en C₀₋₃)-phényle ) et ((alcanediyle en C₀₋₃)- hétéroaryle), le groupe hétéroaryle étant à cinq ou six chaînons et contenant un ou deux hétéroatomes choisis dans le groupe consistant en N, S et 0,
où tous les radicaux alkyle et cycloalkyle mentionnés ci-dessus peuvent être éventuellement substitués par jusqu'à deux radicaux choisis dans le groupe consistant en CF₃, C₂F₅, OH, les radicaux 0-(alkyle en C₁₋₃), NH₂, NH-(alkyle en C₁₋₃), NH- (alcanoyle en C₁₋₃), N(alkyle en C₁₋₃)₂, N(alkyle en C₁₋₃)(alcanoyle en C₁₋₃), COOH, CONH₂ et COO-(alkyle en C₁₋₃), et tous les groupes phényle et hétéroaryle mentionnés ci-dessus peuvent être éventuellement substitués par jusqu'à deux radicaux choisis dans le groupe consistant en F, Cl, Br, CH₃, C₂H₅, OH, OCH₃, OC₂H₅, NO₂, N(CH₃)₂, CF₃, C₂F₅ et SO₂NH₂,
ou R⁵ et R^{5'}, avec l'atome d'azote, forment un noyau hétérocyclique à cinq à sept chaînons, qui peut contenir un atome d'azote ou d'oxygène ou de soufre supplémentaire et qui peut être substitué par un ou des substituants alkyle en C₁₋₄, (alcanediyle en C₀₋₂)-(alcoxy en C₁₋₄), (alcoxy en C₁₋₄)carbonyle, aminocarbonyle ou phényle,
ainsi que leurs formes isomères ou tautomères optiques ou géométriques, ou leurs sels pharmaceutiquement utilisables.

2. Benzimidazoles selon la revendication 1, **caractérisés en ce que**
R¹ est un groupe phényle, qui est éventuellement substitué par jusqu'à deux des substituants ci- après, indépendamment l'un de l'autre :
F, Cl,
OH, OR⁴, OCOR⁴,
SR⁴,
R⁴, ou
deux substituants voisins sur R¹ forment un groupe -O-CH₂-O- ou -CH₂-CH₂-CH₂-.

3. Benzimidazoles selon la revendication 1 ou 2, **caractérisés en ce que**
R² est un groupe hétéroaryle monocyclique à 5-6 chaînons, avec 1-2 hétéroatomes choisis dans le groupe consistant en N, S et 0, qui est éventuellement substitué d'une manière indépendante par jusqu'à deux des substituants suivants :
F, Cl,
OR⁴, OCOR⁴
SR⁴, SOR⁴, SO₂R⁴,
R⁴ ou
deux substituants voisins sur R² forment un groupe -O-CH₂-O- ou CH₂-CH₂-CH₂-.

4. Benzimidazoles selon l'une des revendications 1-3, **caractérisés en ce que** R³ est H.

5. Benzimidazoles selon l'une des revendications 1-4, **caractérisés en ce que**
R⁴ et R⁴' représentent chacun indépendamment de l'autre un radical perfluoralkyle en C₁₋₂, alkyle en C₁₋₄.

6. Benzimidazoles selon l'une des revendications 1-5, **caractérisés en ce que**
R⁵ et R^{5'} représentent chacun indépendamment de l'autre un radical alkyle en C₁₋₆, un atome de carbone pouvant être remplacé par 0, S, SO, SO₂,
un radical (cycloalkyle en C₃₋₅)-(alkylène en C₀₋₃), où, dans un noyau cycloalkyle à cinq chaînons, un chaînon cyclique peut être un atome d'azote ou un atome d'oxygène, l'atome cyclique étant éventuellement substitué par un ou des substituants alkyle en C₁₋₃ ou alcanoyle en C₁₋₃, (alkylène en C₀₋₂)-(hétéroaryle à 5-6 chaînons ayant 1-2 hétéroatomes choisis parmi N, S et 0),
où tous les radicaux alkyle et cycloalkyle mentionnés ci-dessus peuvent être substitués par un ou des substituants CF₃, OH, NH₂, NH (alkyle en C₁₋₃), NH(alcanoyle en C₁₋₃), N(alkyle en C₁₋₃)₂, N(alkyle en C₁₋₃)(alcanoyle en C1-3), COOH, CONH₂, et tous les groupes hétéroaryle mentionnés ci-dessus, ayant un ou deux substituants du groupe consistant en F, Cl, CH₃, C₂H₅, OCH₃, OC₂H₅, CF₃, C₂F₅,
ou R⁵ et R^{5'}, avec l'atome d'azote, forment un radical hétérocyclique à 5-7 chaînons, qui peut contenir un atome d'oxygène, d'azote ou de soufre supplémentaire, et qui peut être substitué par un ou des substituants alkyle en C₁₋₄, (alcoxy en C₁₋₄) - (alkyle en C₀₋₂).

7. Benzimidazoles selon l'une des revendications 1-5, **caractérisés en ce que**
B est COOH ou CONH₂,
chacun étant lié à un atome d'oxygène du groupe A.

8. Composé selon la revendication 1, choisi dans le groupe consistant en
l'acide 5-[[1-(4-méthylphényl)-2-(3-pyridinyl)-1H-benzimidazol-6-yl]oxy]pentanoïque
l'acide 4-[[1-(4-méthylphényl)-2-(3-pyridinyl)-1H-benzimidazol-6-yl]oxy]butyrique
l'acide 5-[[1-(4-méthylphényl)-2-(3-thiényl)-1H-benzimidazol-6-yl]oxy]pentanoïque
l'acide 4-[[1-(4-méthylphényl)-2-(3-thiényl)-1H-benzimidazol-6-yl]oxy]butyrique
l'acide 5-[[1-phényl-2-(3-thiényl)-1H-benzimidazol-6-yl]oxy]pentanoïque
l'acide 4-[[1-phényl-2-(3-thiényl)-1H-benzimidazol-6-yl]oxy]butyrique
l'acide 5-[[1-(4-fluorophényl)-2-(3-thiényl)-1H-benzimidazol-6-yl]oxy]pentanoïque
l'acide 5-[[1-(4-fluorophényl)-2-(3-pyridinyl)-1H-benzimidazol-6-yl]oxy]pentanoïque
l'acide 5-[[1-phényl-2-(3-pyridinyl)-1H-benzimidazol-6-yl]oxy]pentanoïque
l'acide 4-[[1-phényl-2-(3-pyridinyl)-1H-benzimidazol-6-yl]oxy]butyrique
le N-(2-méthoxyéthyl)-5-[[1-(4-méthylphényl)-2-(3-pyridinyl)-1H-benzimidazol-6-yl]oxy]pentanamide
le N,N-diméthyl-5-[[1-(4-méthylphényl)-2-(3-pyridinyl)-1H-benzimidazol-6-yl]oxy]pentanamide
le 5-[[1-(4-méthylphényl)-2-(3-pyridinyl)-1H-benzimidazol-6-yl]oxy]pentanamide.

9. Utilisation d'un composé selon l'une des revendications 1-8 pour la préparation d'un médicament destiné au traitement ou à la prévention de maladies qui sont associées à une activation des microglies.

10. Utilisation selon la revendication 9, pour le traitement ou la prévention de maladies inflammatoires, allergiques, infectieuses ou auto-immunes.

11. Produits pharmaceutiques, qui sont **caractérisés en ce qu'**ils contiennent un ou plusieurs composés selon l'une des revendications 1-8, et un ou plusieurs supports et/ou adjuvants.
